# EUROPÄISCHE PATENTSCHRIFT

(11) **EP 2 062 984 B1**
(45) Veröffentlichungstag und Bekanntmachung des Hinweises auf die Patenterteilung: **23.03.2016**
(21) Anmeldenummer: 09002020.7
(22) Anmeldetag: 07.12.2004
(51) Int. Cl.: C12Q 1/70, G01N 33/53

(54) **Primer und Sonden zum Nachweis genitaler HPV-Genotypen**
Primers and probes for detecting genital hpv genotype
Amorces et sondes pour détecter des génotypes du papillomavirus humain génital

(30) Priorität: 10.12.2003 DE 10357677
(43) Veröffentlichungstag der Anmeldung: 27.05.2009
(62) Teilanmeldung aus: 04803571.1
(73) Patentinhaber: Greiner Bio-One GmbH, 72636 Frickenhausen (DE)
(72) Erfinder: Kranewitter, Wolfgang, 4040 Linz (AT); Mittermayr, Christian, 4111 Walding (AT); Winner, Florian, 4232 Hagenberg (AT); Iftner, Thomas, 72145 Hirrlingen (DE)
(74) Vertreter: Schrell, Andreas

(56) Entgegenhaltungen:
- WO-A-99/63118
- WO-A-03/087829
- DE-A1- 10 009 143
- S-Y CHAN ET AL: "Phylogenetic analysis of 48 papillomavirus types and 28 subtypes and variants: a showcase of the molecular evolution of DNA viruses" JOURNAL OF VIROLOGY, NEW YORK, US, US, Bd. 66, Nr. 10, Oktober 1992 (1992-10), Seiten 5714-5725, XP002094682 ISSN: 0022-538X
- SNIJDERS P J F ET AL: "THE USE OF GENERAL PRIMERS IN THE POLYMERASE CHAIN REACTION PERMITS THE DETECTION OF A BROAD SPECTRUM OF HUMAN PAPILLOMAVIRUS GENOTYPES" JOURNAL OF GENERAL VIROLOGY, READING, BERKS, GB, Bd. 71, Nr. 1, 1990, Seiten 173-181, XP009014549 ISSN: 0022-1317
- TIEBEN L M ET AL: "DETECTION OF CUTANEOUS AND GENITAL HPV TYPES IN CLINICAL SAMPLES BY PCR USING CONSENSUS PRIMERS" JOURNAL OF VIROLOGICAL METHODS, AMSTERDAM, NL, Bd. 2/3, Nr. 42, Mai 1993 (1993-05), Seiten 265-279, XP008004426 ISSN: 0166-0934
- FORSLUND OLA ET AL: "A broad range of human papillomavirus types detected with a general PCR method suitable for analysis of cutaneous tumours and normal skin" JOURNAL OF GENERAL VIROLOGY, Bd. 80, Nr. 9, September 1999 (1999-09), Seiten 2437-2443, XP002326591 ISSN: 0022-1317
- HARWOOD CATHERINE A ET AL: "Degenerate and nested PCR: A highly sensitive and specific method for detection of human papillomavirus infection in cutaneous warts" JOURNAL OF CLINICAL MICROBIOLOGY, Bd. 37, Nr. 11, November 1999 (1999-11), Seiten 3545-3555, XP002326592 ISSN: 0095-1137

### SEQUENZ PROTOKOLL

<110> Greiner Bio-One
<120> Sonden zum Nachweis genitaler HPV-Genotypen
<130> 201888 EP
<140>
   <141>
<160> 135
<170> PatentIn Ver. 2.1
<210> 1
   <211> 25
   <212> DNA
   <213> Humanes Papilloma-Virus
<400> 1
   cargcnaaaw wwktdaarga ytgtg 25
<210> 2
   <211> 25
   <212> DNA
   <213> Humanes Papilloma-Virus
<400> 2
   cargcnaaat atktraaaga ttgtg 25
<210> 3
   <211> 25
   <212> DNA
   <213> Humanes Papilloma-Virus
<400> 3
   cargcaaaat atgtwaagga ttgtg 25
<210> 4
   <211> 25
   <212> DNA
   <213> Humanes Papilloma-Virus
<400> 4
   cargcwaaaa ttgtaaarga ttgtg 25
<210> 5
   <211> 25
   <212> DNA
   <213> Humanes Papilloma-Virus
<400> 5
   caagcaaaaa tagtaaarga ctgtg 25
<210> 6
   <211> 25
   <212> DNA
   <213> Humanes Papilloma-Virus
<400> 6
   cargcaaaat atgtaaaaga ctgtg 25
<210> 7
   <211> 23
   <212> DNA
   <213> Humanes Papilloma-Virus
<400> 7
   aryggytsya rccaaaartg rct 23
<210> 8
   <211> 30
   <212> DNA
   <213> Humanes Papilloma-Virus
<400> 8
   aagctttcta ggaggtacag ttattagtca 30
<210> 9
   <211> 30
   <212> DNA
   <213> Humanes Papilloma-Virus
<400> 9
   ataaaacata ggggttctaa aatagaaggc 30
<210> 10
   <211> 30
   <212> DNA
   <213> Humanes Papilloma-Virus
<400> 10
   actaaagttg acagtgtagg taactggaag 30
<210> 11
   <211> 30
   <212> DNA
   <213> Humanes Papilloma-Virus
<400> 11
   ttaaacaatg gattaagtat aggggtacta 30
<210> 12
   <211> 30
   <212> DNA
   <213> Humanes Papilloma-Virus
<400> 12
   aaaagatgtc tattaaacaa tggattaagt 30
<210> 13
   <211> 29
   <212> DNA
   <213> Humanes Papilloma-Virus
<400> 13
   gggtactaaa gttgacagtg taggtaact 29
<210> 14
   <211> 30
   <212> DNA
   <213> Humanes Papilloma-Virus
<400> 14
   gggaacagtt attagttatg ttaattcctg 30
<210> 15
   <211> 30
   <212> DNA
   <213> Humanes Papilloma-Virus
<400> 15
   aattgtatag ccattgtagg gccacctgac 30
<210> 16
   <211> 30
   <212> DNA
   <213> Humanes Papilloma-Virus
<400> 16
   gggccacctg acactgggaa gtcgtgcttt 30
<210> 17
   <211> 30
   <212> DNA
   <213> Humanes Papilloma-Virus
<400> 17
   gggccacctg acactgggaa gtcgtgcttt 30
<210> 18
   <211> 30
   <212> DNA
   <213> Humanes Papilloma-Virus
<400> 18
   tgatggaggt gattggaagc aaattgttat 30
<210> 19
   <211> 30
   <212> DNA
   <213> Humanes Papilloma-Virus
<400> 19
   tgaaatttct gcaagggtct gtaatatgtt 30
<210> 20
   <211> 30
   <212> DNA
   <213> Humanes Papilloma-Virus
<400> 20
   atggaggtga ttggaagcaa attgttatgt 30
<210> 21
   <211> 30
   <212> DNA
   <213> Humanes Papilloma-Virus
<400> 21
   gaccaatagt gcaattcctg cgataccaac 30
<210> 22
   <211> 30
   <212> DNA
   <213> Humanes Papilloma-Virus
<400> 22
   caaacattat aggcgagccc aaaaacgaca 30
<210> 23
   <211> 30
   <212> DNA
   <213> Humanes Papilloma-Virus
<400> 23
   tcctgcgata ccaacaaata gagtttataa 30
<210> 24
   <211> 28
   <212> DNA
   <213> Humanes Papilloma-Virus
<400> 24
   ctgcgatacc aacaaataga gtttataa 28
<210> 25
   <211> 30
   <212> DNA
   <213> Humanes Papilloma-Virus
<400> 25
   agaaacgatc tatgtgtatg tcacaatggc 30
<210> 26
   <211> 30
   <212> DNA
   <213> Humanes Papilloma-Virus
<400> 26
   agaagagggc gggtcgtgga aggaaattgc 30
<210> 27
   <211> 30
   <212> DNA
   <213> Humanes Papilloma-Virus
<400> 27
   ggcgggtcgt ggaaggaaat tgccaaattt 30
<210> 28
   <211> 30
   <212> DNA
   <213> Humanes Papilloma-Virus
<400> 28
   gcacagaaac gatctatgtg tatgtcacaa 30
<210> 29
   <211> 30
   <212> DNA
   <213> Humanes Papilloma-Virus
<400> 29
   gtagatgtga caaagttagt gacgaaggtg 30
<210> 30
   <211> 30
   <212> DNA
   <213> Humanes Papilloma-Virus
<400> 30
   tgagccttat tagcttttta caaggatgta 30
<210> 31
   <211> 30
   <212> DNA
   <213> Humanes Papilloma-Virus
<400> 31
   caaagttagt gacgaaggtg actggaggga 30
<210> 32
   <211> 30
   <212> DNA
   <213> Humanes Papilloma-Virus
<400> 32
   acagttttta aaagggtgtg ttatatcatg 30
<210> 33
   <211> 30
   <212> DNA
   <213> Humanes Papilloma-Virus
<400> 33
   gggtgtgtta tatcatgtgt aaattctaaa 30
<210> 34
   <211> 30
   <212> DNA
   <213> Humanes Papilloma-Virus
<400> 34
   aaagggtgtg ttatatcatg tgtaaattct 30
<210> 35
   <211> 30
   <212> DNA
   <213> Humanes Papilloma-Virus
<400> 35
   attacacata gatgtgattt aatagatgat 30
<210> 36
   <211> 30
   <212> DNA
   <213> Humanes Papilloma-Virus
<400> 36
   tttaatgcag tttatgcaag gtgtggttat 30
<210> 37
   <211> 30
   <212> DNA
   <213> Humanes Papilloma-Virus
<400> 37
   tgatttaata gatgatggag gaaactggaa 30
<210> 38
   <211> 30
   <212> DNA
   <213> Humanes Papilloma-Virus
<400> 38
   tgcagtttat gcaaggtgtg gttatttcat 30
<210> 39
   <211> 30
   <212> DNA
   <213> Humanes Papilloma-Virus
<400> 39
   ttaatagatg atggaggaaa ctggaaacat 30
<210> 40
   <211> 28
   <212> DNA
   <213> Humanes Papilloma-Virus
<400> 40
   aggtggacga tgacggtgac tggaggga 28
<210> 41
   <211> 30
   <212> DNA
   <213> Humanes Papilloma-Virus
<400> 41
   gcatttctta caaggagcta ttatatccta 30
<210> 42
   <211> 30
   <212> DNA
   <213> Humanes Papilloma-Virus
<400> 42
   aaggcaaatg tccatgtctc aatggataaa 30
<210> 43
   <211> 30
   <212> DNA
   <213> Humanes Papilloma-Virus
<400> 43
   tgtaaacatt acaagcgagc acaaaaaagg 30
<210> 44
   <211> 30
   <212> DNA
   <213> Humanes Papilloma-Virus
<400> 44
   agccttatgc attttttaca gggcacagtt 30
<210> 45
   <211> 30
   <212> DNA
   <213> Humanes Papilloma-Virus
<400> 45
   tatttcatat gtaaactcca ccagccactt 30
<210> 46
   <211> 30
   <212> DNA
   <213> Humanes Papilloma-Virus
<400> 46
   aatttaggtg tagtaaatgt gatgaaggcg 30
<210> 47
   <211> 27
   <212> DNA
   <213> Humanes Papilloma-Virus
<400> 47
   gaagtgtgat gaaggcgact ggaaacc 27
<210> 48
   <211> 30
   <212> DNA
   <213> Humanes Papilloma-Virus
<400> 48
   gaatgagcct gatgcacttt atgcaaggta 30
<210> 49
   <211> 30
   <212> DNA
   <213> Humanes Papilloma-Virus
<400> 49
   cctgatgcac tttatgcaag gtacaataat 30
<210> 50
   <211> 30
   <212> DNA
   <213> Humanes Papilloma-Virus
<400> 50
   tccatgtgca aacactatag gttagcagaa 30
<210> 51
   <211> 30
   <212> DNA
   <213> Humanes Papilloma-Virus
<400> 51
   acaaatgaga cgtatgtcta tgggtgcatg 30
<210> 52
   <211> 30
   <212> DNA
   <213> Humanes Papilloma-Virus
<400> 52
   gacgtatgtc tatgggtgca tggataaaac 30
<210> 53
   <211> 30
   <212> DNA
   <213> Humanes Papilloma-Virus
<400> 53
   cagtaaattt gaagacacag gaaattggaa 30
<210> 54
   <211> 30
   <212> DNA
   <213> Humanes Papilloma-Virus
<400> 54
   ggcactgtaa ttagttatgt aaactccagc 30
<210> 55
   <211> 30
   <212> DNA
   <213> Humanes Papilloma-Virus
<400> 55
   atatgaaaca gtggataaaa tttaggagca 30
<210> 56
   <211> 30
   <212> DNA
   <213> Humanes Papilloma-Virus
<400> 56
   acgccaaatg aatatgtctc aatggattaa 30
<210> 57
   <211> 30
   <212> DNA
   <213> Humanes Papilloma-Virus
<400> 57
   cgccaaatga atatgtctca atggattaaa 30
<210> 58
   <211> 30
   <212> DNA
   <213> Humanes Papilloma-Virus
<400> 58
   tttaagggca ctaaaggaat ttcttaaagg 30
<210> 59
   <211> 30
   <212> DNA
   <213> Humanes Papilloma-Virus
<400> 59
   ggtgcaataa tatcatttgt aaattcaaac 30
<210> 60
   <211> 30
   <212> DNA
   <213> Humanes Papilloma-Virus
<400> 60
   ggcactaaag gaatttctta aaggaacacc 30
<210> 61
   <211> 30
   <212> DNA
   <213> Humanes Papilloma-Virus
<400> 61
   agggcactaa aggaatttct taaaggaaca 30
<210> 62
   <211> 30
   <212> DNA
   <213> Humanes Papilloma-Virus
<400> 62
   cattatctat gtcagcctgg ataaggtata 30
<210> 63
   <211> 30
   <212> DNA
   <213> Humanes Papilloma-Virus
<400> 63
   gcacaaagaa aatcattatc tatgtcagcc 30
<210> 64
   <211> 30
   <212> DNA
   <213> Humanes Papilloma-Virus
<400> 64
   aaatcattat ctatgtcagc ctggataagg 30
<210> 65
   <211> 30
   <212> DNA
   <213> Humanes Papilloma-Virus
<400> 65
   aaacatatga atattggaca atggatacag 30
<210> 66
   <211> 30
   <212> DNA
   <213> Humanes Papilloma-Virus
<400> 66
   gaaagaaaac atatgaatat tggacaatgg 30
<210> 67
   <211> 30
   <212> DNA
   <213> Humanes Papilloma-Virus
<400> 67
   ttaattaggt tcttaagtgg atgtgtaata 30
<210> 68
   <211> 30
   <212> DNA
   <213> Humanes Papilloma-Virus
<400> 68
   taaagcatgt atgtagcaag gtggatgatg 30
<210> 69
   <211> 30
   <212> DNA
   <213> Humanes Papilloma-Virus
<400> 69
   gatgaatatg aaacaatgga taaagcatgt 30
<210> 70
   <211> 30
   <212> DNA
   <213> Humanes Papilloma-Virus
<400> 70
   aagcatgtat gtagcaaggt ggatgatggt 30
<210> 71
   <211> 30
   <212> DNA
   <213> Humanes Papilloma-Virus
<400> 71
   gttatttatg gtcctccaaa cacgggtaaa 30
<210> 72
   <211> 30
   <212> DNA
   <213> Humanes Papilloma-Virus
<400> 72
   aaacaatgga taaagcatgt atgtagcaag 30
<210> 73
   <211> 30
   <212> DNA
   <213> Humanes Papilloma-Virus
<400> 73
   ttagttattt atggtcctcc aaacacgggt 30
<210> 74
   <211> 30
   <212> DNA
   <213> Humanes Papilloma-Virus
<400> 74
   ttagcttttt aggaggtgta gtgctatcat 30
<210> 75
   <211> 30
   <212> DNA
   <213> Humanes Papilloma-Virus
<400> 75
   ttgtgattta gtagaggagg aaggtgagtg 30
<210> 76
   <211> 30
   <212> DNA
   <213> Humanes Papilloma-Virus
<400> 76
   cgttgtgatt tagtagagga ggaaggtgag 30
<210> 77
   <211> 30
   <212> DNA
   <213> Humanes Papilloma-Virus
<400> 77
   catcgttgtg atttagtaga ggaggaaggt 30
<210> 78
   <211> 30
   <212> DNA
   <213> Humanes Papilloma-Virus
<400> 78
   gggcacaaca gcaacaaatg aatatgtgcc 30
<210> 79
   <211> 30
   <212> DNA
   <213> Humanes Papilloma-Virus
<400> 79
   cacaacagca acaaatgaat atgtgccagt 30
<210> 80
   <211> 30
   <212> DNA
   <213> Humanes Papilloma-Virus
<400> 80
   ctgtttggta ctttgtggac cgccaaatac 30
<210> 81
   <211> 30
   <212> DNA
   <213> Humanes Papilloma-Virus
<400> 81
   tttcaagggt ctgtcatttc atttgtgaat 30
<210> 82
   <211> 30
   <212> DNA
   <213> Humanes Papilloma-Virus
<400> 82
   acatttttta aaaggatgca ttatttcata 30
<210> 83
   <211> 30
   <212> DNA
   <213> Humanes Papilloma-Virus
<400> 83
   cgtggtatga caatgggaca atggatacaa 30
<210> 84
   <211> 30
   <212> DNA
   <213> Humanes Papilloma-Virus
<400> 84
   gagcagaaaa gcgtggtatg acaatgggac 30
<210> 85
   <211> 30
   <212> DNA
   <213> Humanes Papilloma-Virus
<400> 85
   aggatgcatt atttcatatg taaattccaa 30
<210> 86
   <211> 30
   <212> DNA
   <213> Humanes Papilloma-Virus
<400> 86
   aaagcgtggt atgacaatgg gacaatggat 30
<210> 87
   <211> 30
   <212> DNA
   <213> Humanes Papilloma-Virus
<400> 87
   agcctgctac attttttaca aggaactgta 30
<210> 88
   <211> 30
   <212> DNA
   <213> Humanes Papilloma-Virus
<400> 88
   gattacacat agaggccgca aggtggcaga 30
<210> 89
   <211> 30
   <212> DNA
   <213> Humanes Papilloma-Virus
<400> 89
   attacacata gaggccgcaa ggtggcagac 30
<210> 90
   <211> 30
   <212> DNA
   <213> Humanes Papilloma-Virus
<400> 90
   agtggattac acatagaggc cgcaaggtgg 30
<210> 91
   <211> 30
   <212> DNA
   <213> Humanes Papilloma-Virus
<400> 91
   acagtggatt acacatagag gccgcaaggt 30
<210> 92
   <211> 30
   <212> DNA
   <213> Humanes Papilloma-Virus
<400> 92
   gtaccattta ttagtgccct taaattgttt 30
<210> 93
   <211> 30
   <212> DNA
   <213> Humanes Papilloma-Virus
<400> 93
   ttacggacca agcgacacag ggaagtcgct 30
<210> 94
   <211> 30
   <212> DNA
   <213> Humanes Papilloma-Virus
<400> 94
   tacggaccaa gcgacacagg gaagtcgcta 30
<210> 95
   <211> 30
   <212> DNA
   <213> Humanes Papilloma-Virus
<400> 95
   gagccttata aattttttcc aagggtcagt 30
<210> 96
   <211> 30
   <212> DNA
   <213> Humanes Papilloma-Virus
<400> 96
   ccttataaat tttttccaag ggtcagtcat 30
<210> 97
   <211> 30
   <212> DNA
   <213> Humanes Papilloma-Virus
<400> 97
   attacaaaag ggcacagcaa cagcaaatga 30
<210> 98
   <211> 30
   <212> DNA
   <213> Humanes Papilloma-Virus
<400> 98
   atggaggaga ctggagaaca atagtaaagc 30
<210> 99
   <211> 30
   <212> DNA
   <213> Humanes Papilloma-Virus
<400> 99
   aagttgtatg gtaatatgcg gaccaccaaa 30
<210> 100
   <211> 30
   <212> DNA
   <213> Humanes Papilloma-Virus
<400> 100
   caaggatgtg taatatcata tgtaaacgcc 30
<210> 101
   <211> 30
   <212> DNA
   <213> Humanes Papilloma-Virus
<400> 101
   aggagactgg agaacaatag taaagctatt 30
<210> 102
   <211> 30
   <212> DNA
   <213> Humanes Papilloma-Virus
<400> 102
   gttgtatggt aatatgcgga ccaccaaaca 30
<210> 103
   <211> 30
   <212> DNA
   <213> Humanes Papilloma-Virus
<400> 103
   aacgacaaat gtcaatgccg caatggatta 30
<210> 104
   <211> 30
   <212> DNA
   <213> Humanes Papilloma-Virus
<400> 104
   cgggcacaaa aacgacaaat gtcaatgccg 30
<210> 105
   <211> 30
   <212> DNA
   <213> Humanes Papilloma-Virus
<400> 105
   tcaatgccgc aatggattaa atttagatgc 30
<210> 106
   <211> 30
   <212> DNA
   <213> Humanes Papilloma-Virus
<400> 106
   atggcgcaat ggattaggtt tagatgtgat 30
<210> 107
   <211> 30
   <212> DNA
   <213> Humanes Papilloma-Virus
<400> 107
   aaatgactat ggcgcaatgg attaggttta 30
<210> 108
   <211> 30
   <212> DNA
   <213> Humanes Papilloma-Virus
<400> 108
   attaggttta gatgtgataa atgtgacgat 30
<210> 109
   <211> 30
   <212> DNA
   <213> Humanes Papilloma-Virus
<400> 109
   taaatttata caaggtgtag ttatttcgta 30
<210> 110
   <211> 30
   <212> DNA
   <213> Humanes Papilloma-Virus
<400> 110
   atgtgattta actaatgatg gtggtaattg 30
<210> 111
   <211> 30
   <212> DNA
   <213> Humanes Papilloma-Virus
<400> 111
   taatgatggt ggtaattgga aagatattgt 30
<210> 112
   <211> 30
   <212> DNA
   <213> Humanes Papilloma-Virus
<400> 112
   taaacaaatg tcaatggcac aatggataca 30
<210> 113
   <211> 30
   <212> DNA
   <213> Humanes Papilloma-Virus
<400> 113
   ttaattagat ttttgcaagg gtgcgttatt 30
<210> 114
   <211> 30
   <212> DNA
   <213> Humanes Papilloma-Virus
<400> 114
   agaaaatcac taacaatgtc agcatggatt 30
<210> 115
   <211> 30
   <212> DNA
   <213> Humanes Papilloma-Virus
<400> 115
   taggtataga tgtgacaaag tgcaagacgg 30
<210> 116
   <211> 30
   <212> DNA
   <213> Humanes Papilloma-Virus
<400> 116
   ttgcgatacc agggtattaa ctttatgtat 30
<210> 117
   <211> 30
   <212> DNA
   <213> Humanes Papilloma-Virus
<400> 117
   gaggtacagt tattagtcat gtaaattcca 30
<210> 118
   <211> 32
   <212> DNA
   <213> Humanes Papilloma-Virus
<400> 118
   taagtttttg gggggaacag ttattagtta tg 32
<210> 119
   <211> 34
   <212> DNA
   <213> Humanes Papilloma-Virus
<400> 119
   ttatacactt tatacaagga gcagtaatat catt 34
<210> 120
   <211> 30
   <212> DNA
   <213> Humanes Papilloma-Virus
<400> 120
   caaggatgta taatatcata tgcaaattca 30
<210> 121
   <211> 35
   <212> DNA
   <213> Humanes Papilloma-Virus
<400> 121
   tgagtttaat aaacttctta gcaggaactg taata 35
<210> 122
   <211> 30
   <212> DNA
   <213> Humanes Papilloma-Virus
<400> 122
   aggcggtacc atattatcat atgtaaatgc 30
<210> 123
   <211> 30
   <212> DNA
   <213> Humanes Papilloma-Virus
<400> 123
   ggaggcactg taattagtta tgtaaactcc 30
<210> 124
   <211> 30
   <212> DNA
   <213> Humanes Papilloma-Virus
<400> 124
   gagttttata catttcctac aaggtgcaat 30
<210> 125
   <211> 30
   <212> DNA
   <213> Humanes Papilloma-Virus
<400> 125
   tatttgcaat gagcctaatg aagtttatgc 30
<210> 126
   <211> 32
   <212> DNA
   <213> Humanes Papilloma-Virus
<400> 126
   gagtttaatt aggttcttaa gtggatgtgt aa 32
<210> 127
   <211> 29
   <212> DNA
   <213> Humanes Papilloma-Virus
<400> 127
   gtttagttat ttatggtcct ccaaacacg 29
<210> 128
   <211> 30
   <212> DNA
   <213> Humanes Papilloma-Virus
<400> 128
   agtcttataa agttttttca agggtctgtc 30
<210> 129
   <211> 32
   <212> DNA
   <213> Humanes Papilloma-Virus
<400> 129
   tgagcctgct acatttttta caaggaactg ta 32
<210> 130
   <211> 27
   <212> DNA
   <213> Humanes Papilloma-Virus
<400> 130
   tgagccttat aaattttttc caagggt 27
<210> 131
   <211> 32
   <212> DNA
   <213> Humanes Papilloma-Virus
<400> 131
   atacatttct tgcaaggcac aataatttca ta 32
<210> 132
   <211> 30
   <212> DNA
   <213> Humanes Papilloma-Virus
<400> 132
   tgagtcttat acatttcttg caaggcacaa 30
<210> 133
   <211> 30
   <212> DNA
   <213> Humanes Papilloma-Virus
<400> 133
   aatgcacttt ttacaaggta cagtaatttc 30
<210> 134
   <211> 32
   <212> DNA
   <213> Humanes Papilloma-Virus
<400> 134
   ttaaatttat acaaggtgta gttatttcgt at 32
<210> 135
   <211> 26
   <212> DNA
   <213> Humanes Papilloma-Virus
<400> 135
   taattagatt tttgcaaggg tgcgtt 26

## Beschreibung

Die vorliegende Erfindung betrifft Oligonucleotide, die als Primer zur Amplifikation von DNA genitaler humaner Papillomaviren (HPV) geeignet sind, Oligonucleotide und Nucleinsäuremoleküle, die als Sonden zum Nachweis und/oder zur Bestimmung genitaler HPV-Genotypen eingesetzt werden können, Verfahren zur Amplifikation der DNA genitaler humaner Papillomaviren, Verfahren zum Nachweis und/oder zur Bestimmung genitaler HPV-Genotypen, die Oligonucleotide und Nucleinsäuremoleküle umfassende Nucleotid-Arrays und Kits sowie die Verwendung der Oligonucleotide und Nucleinsäuremoleküle zur Amplifikation beziehungsweise zum Nachweis und/oder zur Bestimmung genitaler HPV-Genotypen, zur Diagnose und/oder Früherkennung von Krankheiten sowie zur Herstellung von Mitteln zur Diagnose von Krankheiten.

Infektionen mit dem in der Bevölkerung weit verbreiteten humanpathogenen Papilloma-Virus gehören zu den häufigsten sexuell übertragbaren Viruserkrankungen. Die Ansteckung kann jedoch auch beim Neugeborenen über den Geburtsweg erfolgen. Als Folgen einer HPV-Erkrankung treten meist harmlose Hauterscheinungen auf. Bis heute wurden etwa hundert verschiedene Typen dieses Virus nachgewiesen. Papillomaviren werden unterteilt in kutane Typen, die vornehmlich in verhornendem Epithel Läsionen hervorrufen, und Mukosa-Typen, die insbesondere die Schleimhäute infizieren. Eine weitere Unterteilung erfolgt in mit gutartigen Läsionen assoziierten Typen (low risk types; Niedrig-Risiko-Typen) und solchen, die mit präneoplastischen und malignen Epithelveränderungen einhergehen (high risk types; Hoch-Risiko-Typen). Bekannte Hoch-Risiko-Typen sind beilspielsweise die Typen 16, 18, 26, 31, 33, 35, 39, 45, 51, 52, 53, 56, 58, 59, 66, 68, 73, und 82. Bekannte Niedrig-Risiko-HPV-Typen sind beispielsweise die Typen 6, 11, 40, 42, 53, 54, 57 und MM8.

Etwa 25 der Papillomavirus-Typen stehen mit Erkrankungen im Genitalbereich der Frau in ursächlichem Zusammenhang. HPV-Infektionen manifestieren sich im ano-genitalen Bereich der Frau insbesondere in Form kondylomatöser, dysplastischer und neoplastischer Läsionen. Da 99,7 % aller Cervix-Karzinome Papillomavirus-DNA enthalten, wird inzwischen allgemein akzeptiert, dass humane Papilloma-Viren notwendige Risikofaktoren für die Entstehung dieser Krebserkrankung darstellen. Epidemiologische sowie molekulare Studien haben gezeigt, dass eine dauerhafte Infektion mit Hoch-Risiko-HPV-Typen, insbesondere den HPV-Typen 16 und 18, maßgeblich an der Entstehung der Cervix-Karzinoms beteiligt ist.

Sowohl beim Gebärmutterhalskrebs als auch bei anderen Krebserkrankungen, die epidemiologisch mit Papillomaviren assoziiert sind, beispielsweise bestimmten Formen des Hautkrebses, handelt es sich um vermeidbare Erkrankungen, wenn frühzeitige Erkennung und Behandlung gesichert sind. Ein zuverlässiges Verfahren zur Diagnose einer vorliegenden HPV-Infektion ist deshalb Voraussetzung für eine zielgerichtete Therapie.

Diagnostisch sind drei Formen der HPV-Infektion zu unterscheiden, nämlich die klinischen, subklinischen und latenten HPV-Infektionen. HPV-assoziierte Epithelveränderungen des subklinischen beziehungsweise klinisch-manifesten Stadiums der Infektion lassen sich zytologisch relativ gut erfassen. Frühstadien des Cervix-Karzinoms werden daher derzeit hauptsächlich durch Entnahme eines Zellabstriches von der Portio beziehungsweise Cervix uteri in Kombination mit der Kolposkopie erfasst. Obwohl die zytologischen Verfahren dazu beigetragen haben, dass die Inzidenz von Cervix-Karzinomen in den letzten Jahren signifikant gesenkt werden konnte, liefern sie dennoch keine vollkommen befriedigenden Ergebnisse. Eine Prognose über die Weiterentwicklung einzelner Läsionen ist nicht möglich. Die zytologischen Verfahren sind darüber hinaus mit einem relativ großen subjektiven Fehler behaftet und nicht standardisiert.

Da eine Anzucht und kulturelle Vermehrung von HPV nicht möglich ist, beruht der labortechnische HPV-Nachweis insbesondere auf der Erfassung viraler DNA oder Hüllproteine. Mittels immunhistochemischer Färbung lassen sich beispielsweise Gruppen-spezifische Antigene (Kapsidantigene) der Papillomaviren identifizieren. Dieser Test hat jedoch aufgrund seiner geringen Sensitivität nur geringe Aussagekraft. Auch serologische Untersuchungen zum Nachweis HPVspezifischer Antikörper im Serum von Patienten sind ohne Bedeutung für die Diagnostik, da sich selbst bei Patienten mit Cervix-Karzinom nur bei 50% aller Fälle Antikörper nachweisen lassen.

Eine große diagnostische Bedeutung haben Verfahren zum Nachweis viraler Nucleinsäuren in klinischen Gewebeproben. Besondere Bedeutung haben dabei solche Verfahren, die eine Unterscheidung zwischen individuellen Typen einer Niedrig- und Hoch-Risiko-HPV-Infektion ermöglichen, da nur die HPV-Typen HPV16, HPV18, HPV31, HPV33, HPV35, HPV39, HPV45, HPV51, HPV52, HPV56, HPV58, HPV59, HPV66, HPV68 und HPV82 mit der Entwicklung von Cervix-Karzinomen in situ assoziiert sind, wobei wahrscheinlich auch HPV53 karzinogen ist.

Ein bekanntes Verfahren zum Nachweis von HPV-Nucleinsäuren ist das HCM (Hybrid Capture Microplate)-Verfahren der Firma Digene (HC2 HPV DNA-Test), dem ein signalverstärkendes Hybridisierungsverfahren zugrunde liegt. Als Hybridisierungssonden werden HPVspezifische RNA-Sequenzen eingesetzt. Nach Inkubation der Sonden mit denaturierter HPV-DNA aus infiziertem Gewebe werden die gebildeten RNA/DNA-Hybride über spezifische Antikörper an der Mikroplatten-Oberfläche einer Mikroplatte gebunden. Der Nachweis der RNA/DNA-Hybride erfolgt über einen zweiten Antikörper, der mit alkalischer Phosphatase markiert ist. Dieses Enzym kann nach Zugabe bestimmter Substanzen messbares Licht erzeugen. Das HCM-Verfahren erlaubt eine subgruppen-spezifische Differenzierung zwischen den low risk-Typen 6, 11, 42, 43 und 44 und den high risk-Typen 16, 18, 31, 33, 35, 39, 45, 51, 52, 56, 58, 59 und 68 und kann daher bei der Differentialdiagnostik unklarer zytologischer Befunde hilfreich sein. Ein Nachteil dieses Verfahrens ist allerdings, dass einige high risk-Typen nicht erfasst werden und dass es darüber hinaus zu einer Kreuzreaktion zwischen beiden Subgruppen kommt.

Viele im Stand der Technik bekannten Verfahren zum Nachweis viraler Nucleinsäuren in klinischen Gewebeproben beruhen auf vorausgehender HPV-Genamplifikation. Als sensitivstes Verfahren hat sich dabei das PCR-Verfahren erwiesen. Mittels derartiger Verfahren kann ein relativ breites Spektrum von HPV-Viren nachgewiesen und anschließend auch einer Typisierung unterworfen werden, wobei die Typisierung hauptsächlich mittels Sequenz-Analyse des PCR-Amplifikationsproduktes erfolgt. Die Typisierung erlaubt eine genaue Charakterisierung des HPV-Typs nicht nur bei Einzel-, sondern auch bei Mehrfach- beziehungsweise Mischinfektionen und ermöglicht so eine über den zytologischen Befund hinausgehende Beurteilung des onkogenen Potentials der nachgewiesenen HPV-Genotypen.

Snijders et al. J. Gen. Virol., 71 (1990), 173-181, und Surentheran et al., J. Clin. Path., 51 (1998), 606-610, beschreiben PCR-Verfahren zum Nachweis von HPV-DNA, wobei Primer-Paare eingesetzt werden, die innerhalb des HPV-Strukturgens L1 liegen. Das amplifizierte Genfragment wird dann anschließend sequenziert, um eine Typisierung der nachgewiesenen HPV-Typen vorzunehmen. Nachteilig bei beiden Verfahren ist allerdings, dass das L1-Gen weniger gut konserviert ist als andere Bereiche der HPV-DNA. Mittels der beschriebenen Verfahren kann daher nur eine begrenzte Anzahl von HPV-Typen erfasst werden. Beispielsweise lassen sich mittels der von Snijder et al. beschriebenen Primer einige HPV-Typen wie HPV30, HPV39 und HPV51 nur mit stark verminderter Sensitivität nachweisen. Darüber hinaus ergeben einige HPV-Typen wie HPV18 bei Verwendung der von Snijder et al. beschriebenen Primer zusätzliche Banden.

Die DE 100 09 143 A1 beschreibt ein PCR-Verfahren zum Nachweis einer allgemeinen HPV-Infektion, insbesondere jedoch von HPV-DNA im Anogenitalbereich, wobei 2 Primer-Paare eingesetzt werden, die innerhalb des konservierten HPV-Gens E1 liegen. Allerdings lassen sich nur die unter Verwendung eines Primer-Paares erhaltenen Amplifikationsprodukte zur sicheren Typisierung einsetzen, nicht jedoch die unter Verwendung des zweiten Primer-Paares erhaltenen Amplifikationsprodukte. Nachteilig ist ferner, dass die Typisierung erfolgt, indem die Amplifikationsprodukte entweder sequenziert oder mittels Temperaturgradienten-Gelelektrophorese untersucht werden müssen. Sowohl die Sequenzierung als auch die Durchführung einer Temperaturgradienten-Gelelektrophorese sind arbeits- und kostenintensive Verfahren.

Ein erheblicher Nachteil der bekannten kommerziellen Tests zum Nachweis und zur Typisierung von HPV-Genotypen besteht daher vor allem darin, dass diese ein nur eingeschränktes HPV-Typenspektrum nachweisen, wobei insbesondere seltene genitale HPV-Typen nur unzureichend erfasst werden, obwohl ein hohes onkogenes Potential einiger dieser Virustypen bekannt ist. Nachteilig ist ferner, dass nach der Amplifikation in jedem Fall eine zeit- und kostenintensive Sequenzierung durchgeführt werden muss, um eine Typisierung vornehmen zu können.

Das der vorliegenden Erfindung zugrunde liegende technische Problem besteht in der Bereitstellung von Mitteln und Verfahren, die einen schnellen und zweifelsfreien Nachweis beziehungsweise eine einfache und schnelle Typisierung insbesondere genitaler Hoch-Risiko-HPV-Genotypen erlauben und die die im Stand der Technik bekannten Nachteile nicht aufweisen, das heißt, insbesondere alle die HPV-Typen erfassen, von denen bekannt ist, dass sie mit Krebserkrankungen der Frau assoziiert sind.

Die vorliegende Erfindung löst das ihr zugrunde liegende technische Problem durch die Bereitstellung von Oligonucleotiden, die als Forward-Primer und Reverse-Primer zur Amplifikation eines Nucleinsäure-Bereiches eines genitalen humanen Papillomavirus (HPV) eingesetzt werden können. Die vorliegende Erfindung löst das ihr zugrunde liegende technische Problem insbesondere durch die Bereitstellung eines Oligonucleotides, das als Forward-Primer zur Amplifikation eines Nucleinsäure-Bereiches eines genitalen humanen Papillomavirus (HPV) eingesetzt werden kann, und das die Sequenz 5'-CAR GCI AAA WWW KTD AAR GAY TGT G-3' oder 5'-CAR GCN AAA WWW KTD AAR GAY TGT G-3' (SEQ ID Nr. 1) aufweist, wobei R = A oder G ist, W = T oder A ist, K = T oder G ist, I = Inosin ist, N = A, T, G oder C ist, D = A, T oder G ist und Y = C oder T ist.

Das als Forward-Primer eingesetzte erfindungsgemäße Oligonucleotid ist vorzugsweise ausgewählt aus der Gruppe bestehend aus:
a) einem Oligonucleotid mit der Sequenz 5'-CAR GCI AAA TAT KTR AAA GAT TGT G-3' oder 5'-CAR GCN AAA TAT KTR AAA GAT TGT G-3' (SEQ ID Nr. 2),
b) einem Oligonucleotid mit der Sequenz 5'-CAR GCA AAA TAT GTW AAG GAT TGT G-3' (SEQ ID Nr. 3),
c) einem Oligonucleotid mit der Sequenz 5'-CAR GCW AAA ATT GTA AAR GAT TGT G-3' (SEQ ID Nr. 4),
d) einem Oligonucleotid mit der Sequenz 5'-CAA GCA AAA ATA GTA AAR GAC TGT G-3' (SEQ ID Nr. 5) und
e) einem Oligonucleotid mit der Sequenz 5'-CAR GCA AAA TAT GTA AAA GAC TGT G-3' (SEQ ID Nr. 6),
wobei R = A oder G ist, W = T oder A ist, K = T oder G ist, I = Inosin ist und N = A, T, G oder C ist.

In besonders bevorzugter Ausführungsform der Erfindung wird als Forward-Primer zur Amplifikation der Nucleinsäure genitaler humaner Papillomaviren ein äquimolares Gemisch der Oligonucleotide mit den in SEQ ID Nr. 2 bis 6 dargestellten Nucleotidsequenzen eingesetzt.

Die vorliegende Erfindung löst das ihr zugrunde liegende technische Problem auch durch die Bereitstellung eines Gemischs aus Oligonucleotiden, das als Primer, insbesondere als Reverse-Primer, zur Amplifikation eines Nucleinsäure-Bereiches eines genitalen humanen Papillomavirus eingesetzt werden kann, wobei das Oligonucleotid die Sequenz 5'-ARY GGY TSY ARC CAA AAR TGR CT-3' (SEQ ID Nr. 7) aufweist, wobei R = A oder G ist, Y = C oder T ist und S = C oder G ist.

Die vorliegende Offenbarung löst das ihr zugrunde liegende technische Problem auch durch die Bereitstellung eines Oligonucleotides, das als Sonde zum Nachweis und/oder zur Bestimmung genitaler HPV-Genotypen eingesetzt werden kann, ausgewählt aus der Gruppe bestehend aus:
1) einem Oligonucleotid mit der in SEQ ID Nr. 8, 9 oder 117 dargestellten Nucleotidsequenz zum Nachweis und/oder zur Bestimmung des HPV6-Genotyps, insbesondere des HPV6b-Genotyps,
2) einem Oligonucleotid mit der in SEQ ID Nr. 10, 11, 12, 13, 14, 15, 16, 17 oder 118 dargestellten Nucleotidsequenz zum Nachweis und/oder zur Bestimmung des HPV11-Genotyps,
3) einem Oligonucleotid mit der in SEQ ID Nr. 18, 19 oder 20 dargestellten Nucleotidsequenz zum Nachweis und/oder zur Bestimmung des HPV16-Genotyps,
4) einem Oligonucleotid mit der in SEQ ID Nr. 21, 22, 23, 24 oder 119 dargestellten Nucleotidsequenz zum Nachweis und/oder zur Bestimmung des HPV18-Genotyps,
5) einem Oligonucleotid mit der in SEQ ID Nr. 25, 26, 27 oder 28 dargestellten Nucleotidsequenz zum Nachweis und/oder zur Bestimmung des HPV26-Genotyps,
6) einem Oligonucleotid mit der in SEQ ID Nr. 29, 30, 31 oder 120 dargestellten Nucleotidsequenz zum Nachweis und/oder zur Bestimmung des HPV31-Genotyps,
7) einem Oligonucleotid mit der in SEQ ID Nr. 32, 33 oder 34 dargestellten Nucleotidsequenz zum Nachweis und/oder zur Bestimmung des HPV33-Genotyps,
8) einem Oligonucleotid mit der in SEQ ID Nr. 35, 36, 37, 38 oder 39 dargestellten Nucleotidsequenz zum Nachweis und/oder zur Bestimmung des HPV34-Genotyps,
9) einem Oligonucleotid mit der in SEQ ID Nr. 40 oder 41 dargestellten Nucleotidsequenz zum Nachweis und/oder zur Bestimmung des HPV35-Genotyps, insbesondere des HPV35h-Genotyps,
10) einem Oligonucleotid mit der in SEQ ID Nr. 42, 43, 44, 45 oder 46 dargestellten Nucleotidsequenz zum Nachweis und/oder zur Bestimmung HPV39-Genotyps,
11) einem Oligonucleotid mit der in SEQ ID Nr. 47, 48, 49 oder 50 dargestellten Nucleotidsequenz zum Nachweis und/oder zur Bestimmung des HPV40-Genotyps,
12) einem Oligonucleotid mit der in SEQ ID Nr. 51, 52 oder 121 dargestellten Nucleotidsequenz zum Nachweis und/oder zur Bestimmung des HPV42-Genotyps,
13) einem Oligonucleotid mit der in SEQ ID Nr. 122 dargestellten Nucleotidsequenz zum Nachweis und/oder zur Bestimmung des HPV43-Genotyps,
14) einem Oligonucleotid mit der in SEQ ID Nr. 53, 54, 55 oder 123 dargestellten Nucleotidsequenz zum Nachweis und/oder zur Bestimmung des HPV44-Genotyps,
15) einem Oligonucleotid mit der in SEQ ID Nr. 56, 57, 58, 59, 60, 61 oder 124 dargestellten Nucleotidsequenz zum Nachweis und/oder zur Bestimmung des HPV45-Genotyps,
16) einem Oligonucleotid mit der in SEQ ID Nr. 62, 63, 64 oder 125 dargestellten Nucleotidsequenz zum Nachweis und/oder zur Bestimmung des HPV51-Genotyps,
17) einem Oligonucleotid mit der in SEQ ID Nr. 65, 66, 67 oder 126 dargestellten Nucleotidsequenz zum Nachweis und/oder zur Bestimmung des HPV52-Genotyps,
18) einem Oligonucleotid mit der in SEQ ID Nr. 68, 69, 70, 71, 72, 73 oder 127 dargestellten Nucleotidsequenz zum Nachweis und/oder zur Bestimmung des HPV53-Genotyps,
19) einem Oligonucleotid mit der in SEQ ID Nr. 74, 75, 76 oder 77 dargestellten Nucleotidsequenz zum Nachweis und/oder zur Bestimmung des HPV54-Genotyps,
20) einem Oligonucleotid mit der in SEQ ID Nr. 78, 79, 80, 81 oder 128 dargestellten Nucleotidsequenz zum Nachweis und/oder zur Bestimmung des HPV56-Genotyps,
21) einem Oligonucleotid mit der in SEQ ID Nr. 82, 83, 84, 85 oder 86 dargestellten Nucleotidsequenz zum Nachweis und/oder zur Bestimmung des HPV58-Genotyps,
22) einem Oligonucleotid mit der in SEQ ID Nr. 87 oder 129 dargestellten Nucleotidsequenz zum Nachweis und/oder zur Bestimmung des HPV59-Genotyps,
23) einem Oligonucleotid mit der in SEQ ID Nr. 88, 89, 90, 91, 92, 93 oder 94 dargestellten Nucleotidsequnz zum Nachweis und/oder zur Bestimmung des HPV61-Genotyps,
24) einem Oligonucleotid mit der in SEQ ID Nr. 95, 96, 97 oder 130 dargestellten Nucleotidsequenz zum Nachweis und/oder zur Bestimmung des HPV66-Genotyps,
25) einem Oligonucleotid mit der in SEQ ID Nr. 98, 99, 100, 101 oder 102 dargestellten Nucleotidsequenz zum Nachweis und/oder zur Bestimmung des HPV67-Genotyps,
26) einem Oligonucleotid mit der in SEQ ID Nr. 103, 104, 105, 131 oder 132 dargestellten Nucleotidsequenz zum Nachweis und/oder zur Bestimmung des HPV68-Genotyps,
27) einem Oligonucleotid mit der in SEQ ID Nr. 106, 107, 108 oder 133 dargestellten Nucleotidsequenz zum Nachweis und/oder zur Bestimmung des HPV70-Genotyps,
28) einem Oligonucleotid mit der in SEQ ID Nr. 109, 110, 111, 112 oder 134 dargestellten Nucleotidsequenz zum Nachweis und/oder zur Bestimmung des HPV73-Genotyps, und
29) einem Oligonucleotid mit der in SEQ ID Nr. 113, 114, 115, 116 oder 135 dargestellten Nucleotidsequenz zum Nachweis und/oder zur Bestimmung des HPV82-Genotyps.

Eine besonders bevorzugte Ausführungsform betrifft die Bereitstellung eines Oligonucleotides, das als Sonde zum Nachweis und/oder zur Bestimmung genitaler HPV-Genotypen eingesetzt werden kann, ausgewählt aus den Oligonucleotiden mit einer der in SEQ ID Nr. 19, SEQ ID Nr. 32, SEQ ID Nr. 41, SEQ ID Nr. 44, SEQ ID Nr. 48, SEQ ID Nr. 82 oder SEQ ID Nr. 117 bis SEQ ID Nr. 135 dargestellten Nucleotidsequenzen.

Die erfindungsgemäßen Oligonucleotide mit den in SEQ ID Nr. 1 bis 7 dargestellten Nucleotidsequenzen, erlauben in vorteilhafter Weise eine äußerst spezifische Amplifikation von Nucleinsäure-Bereichen bei einer Vielzahl unterschiedlicher humaner HPV-Typen, wobei erfindungsgemäß insbesondere vorgesehen ist, dass ein spezifischer Bereich des HPV-Gens E1 amplifiziert wird. Das E1-Gen als Zielbereich zur Amplifikation und damit zum Nachweis von HPV-Typen bietet gegenüber anderen Bereichen des HPV-Genoms eine Reihe erheblicher Vorteile.

Das ringförmige HPV-Genom mit einer Größe von etwa 7,9 kB besteht aus den Genen E6, E7, E1, E2, E4, E5, L2, L1 und der "long control region" (LCR), wobei die Gene in dieser Reihenfolge auf dem Genom angeordnet sind. Bei HPV-Infektionen kommt es sehr häufig zu einer Integration des Virus in das humane Genom, wobei in vielen Fällen Teile des Virus-Genoms deletiert werden. So ist bekannt, dass beispielsweise in Karzinomen die Gene E2, E4, E5 und L2 zumindest teilweise deletiert werden können. Die Gene E2, E4, E5 und L2 kommen somit als Zielregion zur Amplifikation nicht in Frage, da ein gesicherter HPV-DNA-Nachweis in einer Probe nicht möglich ist. Hingegen ist bekannt, dass E1 bei Integration des Virus in das humane Genom sehr häufig erhalten bleibt, wobei E1 zumindest weit weniger häufig als das L1-Gen deletiert wird. Ob E1 allerdings immer erhalten bleibt, ist derzeit noch nicht völlig geklärt. E1 wird jedoch mit großer Wahrscheinlichkeit auch bei Integration des Virus in das humane Genom amplifiziert und kann deshalb bei sehr vielen HPV-Infektionen nachgewiesen werden. Unter Verwendung der erfindungsgemäßen Oligonucleotid-Primer können daher insbesondere auch persistierende HPV-Infektionen erfasst werden, bei denen das Risiko der Krebsentwicklung sehr groß ist.

Obwohl auch der LCR-Bereich und die Gene E6 und E7 bei Integration des Virus in das humane Genom erhalten bleiben und nicht deletiert werden, sind auch diese Genom-Bereiche als Zielregion zur Amplifikation von HPV-DNA nicht geeignet, da die Sequenzen dieser Bereiche bei den verschiedenen HPV-Typen teilweise stark divergieren können. Die Verwendung dieser Genom-Bereiche als Zielregion zur Amplifikation würde eine Vielzahl unterschiedlicher Primer-Paare erfordern, um alle HPV-Typen zu erfassen. Auch vom L1-Gen ist bekannt, dass seine Sequenz bei den einzelnen HPV-Typen weniger konserviert ist als andere HPV-Genombereiche, so dass nicht alle HPV-Typen erfasst werden können. Im Gegensatz dazu weist das E1-Gen den Vorteil auf, dass es bei den unterschiedlichen HPV-Typen relativ gut konserviert ist, so dass bei Amplifikation des E1-Gens ein äußerst breites Spektrum unterschiedlicher HPV-Typen erfasst wird. Minimale Sequenzunterschiede zwischen einzelnen HPV-Typen werden erfindungsgemäß insbesondere dadurch kompensiert, dass in bevorzugter Ausführungsform der Erfindung ein äquimolares Gemisch der Oligonucleotide mit den in SEQ ID Nr. 2 bis 6 dargestellten Nucelotidsequenzen, als Forward-Primer eingesetzt wird, so dass mittels einer einzigen Amplifikationsreaktion bei allen HPV-Typen ein Amplifikationsprodukt erhalten und somit ein Nachweis aller HPV-Typen ermöglicht wird.

Erfindungsgemäß ist vorgesehen, dass das unter Verwendung der erfindungsgemäßen Forward- und Reverse-Primer erhaltene Amplifikationsprodukt dann unter Verwendung der erfindungsgemäßen Oligonucleotid-Sonden nachgewiesen wird. Die erfindungsgemäß als Sonden eingesetzten Oligonucleotide erlauben in äußerst vorteilhafter Weise den gezielten Nachweis individueller HPV-Typen und somit eine Typisierung der HPV-Typen in einer biologischen Probe. Unter Verwendung der erfindungsgemäßen Oligonucleotid-Sonden lassen sich mehr als zwanzig sehr häufig auftretende genitalpathogene HPV-Typen nachweisen und unterscheiden. In einer besonders bevorzugten Ausführungsform der Erfindung sind die erfindungsgemäßen Oligonucleotid-Sonden auf einem Nucleotid-Array enthalten, so dass eine Vielzahl von Amplifikationsprodukten in kurzer Zeit auf einfache Art und Weise getestet und eine Typisierung der HPV-Genotypen vorgenommen werden kann. Die erfindungsgemäßen Oligonucleotide erlauben somit eine äußerst effiziente und schnelle Typisierung von Papillomaviren, während die im Stand der Technik bekannten Typisierungs-Verfahren zeitaufwändige und kostenintensive Sequenz-Analysen oder Gelelektrophoresen erfordern. Erfindungsgemäß ist insbesondere vorgesehen, die erfindungsgemäßen Oligonucleotide beziehungsweise die unter Verwendung dieser Oligonucleotide durchgeführten erfindungsgemäßen Verfahren zur Amplifikation beziehungsweise zum Nachweis und zur Typisierung von humanen Papilloma-Viren insbesondere in solchen Fällen einzusetzen, in denen bereits vorher eine HPV-Infektion festgestellt und nachgewiesen wurde. Erfindungsgemäß ist also vorgesehen, die erfindungsgemäßen Oligonucleotide und Verfahren primär nicht zum Screenen auf HPV-Infektionen einzusetzen, sondern zur Typisierung, d.h. zum Nachweis und zur Bestimmung individueller HPV-Genotypen. Zur Feststellung beziehungsweise zum Nachweis der HPV-Infektion kann ein herkömmlicher Test, beispielsweise der Hybrid Capture-Test von Digene eingesetzt werden. Mit Hilfe des Hybrid Capture-Tests lassen sich Low- von High Risk-Typen unterscheiden. Da dieser Test sehr häufig zur Untersuchung von High Risk-Typen eingesetzt wird, ist zu erwarten, dass mit diesem Test hauptsächlich die klinisch relevanten High Risk-Typen erfasst werden, die dann unter Verwendung der erfindungsgemäßen Oligonucleotide beziehungsweise der erfindungsgemäßen Verfahren typisiert werden können.

Im Zusammenhang mit der vorliegenden Erfindung wird unter einem "Oligonucleotid" ein isoliertes und gereinigtes Molekül verstanden, das aus zwei bis weniger als einhundert Nucleotiden besteht. Bei den erfindungsgemäßen Oligonucleotiden kann es sich um, vorzugsweise gereinigte und isolierte, DNA-, RNA-, PNA- oder LNA-Moleküle oder Mischformen davon handeln.

Bei "PNA" ("Peptide Nucleic Acid" oder "Polyamide Nucleic Acid")-Sequenzen handelt es sich um Moleküle, die nicht negativ geladen sind und in gleicher Weise wie DNA wirken (Nielsen et al., Science, 254 (1991), 1497-1500; Nielsen et al., Biochemiestry, 26 (1997), 5072-5077; Weiler et al., Nuc. Acids Res., 25 (1997), 2792-2799). PNA-Sequenzen umfassen ein Polyamid-Grundgerüst aus N-(2-Aminoethyl)-glycin-Einheiten und besitzen keine Glucose-Einheiten und keine Phosphat-Gruppen. Die unterschiedlichen Basen sind über Methylen-Carbonyl-Bindungen an das Grundgerüst gebunden. "LNA" (Locked Nucleic Acid)-Moleküle sind dadurch gekennzeichnet, dass die Furanose-Ring-Konformation durch einen Methylen-Linker beschränkt ist, der die 2'-O-Position mit der 4'-C-Position verbindet. LNAs werden als einzelne Nucleotide in Nucleinsäuren, beispielsweise DNA- oder RNA, eingebaut. LNA-Oligonucleotide unterliegen ebenfalls wie PNA-Moleküle den Watson-Crick-Basenpaarungs-Regeln und hybridisieren an komplementäre Oligonucleotide. LNA/DNA- oder LNA/RNA-Duplexmoleküle zeigen gegenüber ähnlichen Duplexmolekülen, die ausschließlich aus DNA oder RNA gebildet werden, erhöhte thermische Stabilität.

Die Oligonucleotide mit den in SEQ ID Nr. 1 bis 7 dargestellten Nucleotidsequenzen sind, komplementär zu Konsensus-Sequenzen die im E1-Genbereich von mindestens 29 genitalen HPV-Genotypen vorhanden sind, so dass unter Verwendung dieser Oligonucleotide als Forward- und/oder Reverse-Primer eine Amplifikation des E1-Genbereiches aller dieser 29 genitalen HPV-Genotypen möglich ist. Die Oligonucleotide mit den in SEQ ID Nr. 8 bis 135 dargestellten Nucleotidsequenzen, insbesondere die Oligonucleotide mit den in SEQ ID Nr. 19, 32, 41, 44, 48, 82 und 117 bis 135 dargestellten Sequenzen, sind hingegen nur zu Sequenzen jeweils eines bestimmten genitalen HPV-Genotyps komplementär, jedoch keine Komplementarität zu den Sequenzen anderer genitaler HPV-Genotypen aufweisen, so dass die Verwendung eines der Oligonucleotide mit den in SEQ ID Nr. 8 bis 135 dargestellten Nucleotidsequenzen, insbesondere eines der Oligonucleotide mit den in SEQ ID Nr. 19, 32, 41, 44, 48, 82 und 117 bis 135 dargestellten Sequenzen, den spezifischen Nachweis eines bestimmten genitalen HPV-Genotyps ermöglicht. Im Zusammenhang mit der vorliegenden Erfindung ist eine Nucleinsäure zu einer anderen Nucleinsäure "komplementar", wenn sie mit dieser über ihre gesamte Länge oder zumindest über den größten Teil ihrer Länge hinweg einen Doppelstrang bilden kann, in dem alle Nucleotide nach den Regeln von Watson und Crick durch Wasserstoff-Brückenbindung gepaart vorliegen.

In einem weiteren Aspekt der Offenbarung weisen die offenbarten Oligonucleotide eine Nucleotidsequenz auf, die gegenüber einer der in SEQ ID Nr. 1 bis 135 dargestellten Nucleotidsequenzen, insbesondere gegenüber einer der in SEQ ID Nr. 1 bis 7, 19, 32, 41, 44, 48, 82 oder 117 bis 135 dargestellten Nucleotidsequenzen mutiert ist, wobei die Oligonucleotide erhältlich sind durch:
a) Deletion von 1 bis 10 Nucleotiden in einer der in SEQ ID Nr. 1 bis 135 dargestellten Nucleotidsequenzen, insbesondere in einer der in SEQ ID Nr. 1 bis 7, 19, 32, 41, 44, 48, 82 oder 117 bis 135 dargestellten Nucleotidsequenzen,
b) Addition von 1 bis 10 Nucleotiden in einer der in SEQ ID Nr. 1 bis 135 dargestellten Nucleotidsequenzen, insbesondere in einer der in SEQ ID Nr. 1 bis 7, 19, 32, 41, 44, 48, 82 oder 117 bis 135 dargestellten Nucleotidsequenzen, und/oder
c) Substitution von 1 bis 3 Nucleotiden in einer der in SEQ ID Nr. 1 bis 135 dargestellten Nucleotidsequenzen, insbesondere in einer der in SEQ ID Nr. 1 bis 7, 19, 32, 41, 44, 48, 82 oder 117 bis 135 dargestellten Nucleotidsequenzen.

Bevorzugt liegt die Deletion oder Addition der Nucleotide am 5'-Ende und/oder 3'-Ende einer der in SEQ ID Nr. 1 bis 135 dargestellten Nucleotidsequenzen vor. Im Falle einer Addition ist erfindungsgemäß ferner bevorzugt, dass die zusätzlichen Nucleotide zusammen mit einer der in SEQ ID Nr. 1 bis 135 dargestellten Nucleotidsequenzen eine Sequenz bilden, die über ihre gesamte Länge oder zumindest über den größten Teil ihrer Länge hinweg zu den entsprechenden Zielsequenzen im E1-Genbereich komplementär ist.

Die vorliegende Offenbarung umfasst selbstverständlich auch Oligonucleotide, die eine Nucleotidsequenz aufweisen, die über ihre gesamte Länge hinweg zu einer der in SEQ ID Nr. 1 bis 135 dargestellten Nucleotidsequenzen, insbesondere zu einer der in SEQ ID Nr. 1 bis 7, 19, 32, 41, 44, 48, 82 oder 117 bis 135 dargestellten Nucleotidsequenzen, oder zu einer der erfindungsgemäßen mutierten Nucleotidsequenzen, die dadurch charakterisiert sind, dass sie gegenüber einer der in SEQ ID Nr. 1 bis 135 dargestellten Nucleotidsequenzen, insbesondere einer der in SEQ ID Nr. 1 bis 7, 19, 32, 41, 44, 48, 82 oder 117 bis 135 dargestellten Nucleotidsequenzen, eine Addition und/oder Deletion von 1 bis 10 Nucleotiden und/oder eine Substitution von 1-3 Nucleotiden aufweisen, komplementär ist.

Die vorliegende Erfindung löst das ihr zugrunde liegende technische Problem auch durch die Bereitstellung eines Primer-Paars zur Amplifikation eines Nucleinsäure-Bereiches eines genitalen humanen Papilloma-Virus, umfassend einen Forward-Primer und einen Reverse-Primer, wobei der Forward-Primer ausgewählt ist aus der Gruppe bestehend aus:
a) einem Oligonucleotid mit einer der in SEQ ID Nr. 1 bis 6 dargestellten Nucleotidsequenzen,
b) einem Gemisch der Oligonucleotide nach a) und der Reverse-Primer ausgewählt ist aus der Gruppe bestehend aus:
   einem Oligonucleotid mit der in SEQ ID Nr. 7 dargestellten Nucleotidsequenz,

In einer bevorzugten Ausführungsform umfasst das Primer-Paar ein äquimolares Gemisch der Oligonucleotide mit den in SEQ ID Nr. 2 bis 6 dargestellten Nucleotidsequenzen als Forward-Primer und das Oligonucleotid mit der in SEQ ID Nr. 7 dargestellten Nucelotidsequenz als Reverse-Primer.

In einem weiteren Aspekt der Offenbarung ist vorgesehen, dass Oligonucleotide mit einer der in SEQ ID Nr. 8 bis 135 dargestellten Nucleotidsequenzen, insbesondere einer der in SEQ ID Nr. 19, 32, 41, 44, 48, 82 oder 117 bis 135 dargestellten Nucleotidsequenzen, einer mutierten Nucleotidsequenz davon oder einer komplementären Sequenz davon Teil eines längeren Oligonucleotids oder Polynucleotids sein können. Das längere Oligonucleotid oder Polynucleotid, das im Folgenden auch als Nucleinsäuremolekül bezeichnet wird, umfasst daher neben einer der in SEQ ID Nr. 8 bis 135 dargestellten Nucleotidsequenzen, insbesondere einer der in SEQ ID Nr. 19, 32, 41, 44, 48, 82 oder 117 bis 135 dargestellten Nucleotidsequenzen, einer mutierten Sequenz davon oder einer komplementären Sequenz weitere zusätzliche Nucleotide und/oder Nucleotidsequenzen. Im Zusammenhang mit der vorliegenden Erfindung wird unter einem "Polynucleotid" ein gereinigtes und isoliertes Molekül verstanden, das aus mindestens einhundert Nucleotiden besteht.

Die vorliegende Offenbarung betrifft daher auch ein, vorzugsweise gereinigtes und isoliertes Nucleinsäuremolekül, umfassend mindestens einen Bereich, der eine der in SEQ ID Nr. 8 bis 135 dargestellten Nucleotidsequenzen, insbesondere eine der in SEQ ID Nr. 1 bis 7, 19, 32, 41, 44, 48, 82 oder 117 bis 135 dargestellten Nucleotidsequenzen, eine mutierte Nucleotidsequenz davon, erhältlich durch Deletion und/oder Addition von 1 bis 10 Nucleotiden und/oder Substitution von 1 bis 3 Nucleotiden einer der in SEQ ID Nr. 8 bis 135 dargestellten Nucleotidsequenzen, insbesondere einer der in SEQ ID Nr. 1 bis 7, 19, 32, 41, 44, 48, 82 oder 117 bis 135 dargestellten Nucleotidsequenzen, oder eine zu diesen Sequenzen komplementäre Nucleotidsequenz aufweist, und einen oder mehr zusätzliche Bereiche mit einer Gesamtlänge von mindestens 1 Nucleotid. Bei dem Nucleinsäuremolekül kann es sich um ein längeres Oligonucleotid oder ein Polynucleotid handeln. Wenn das Nucleinsäuremolekül als längeres Oligonucleotid vorliegt, beträgt die Gesamtlänge des erfindungsgemäßen längeren Oligonucleotids höchstens 99 Nucleotide. Wenn das längere Oligonucleotid beispielsweise eine der in SEQ ID Nr. 8 bis 135 dargestellten Nucleotidsequenzen umfasst, können die zusätzlichen Bereiche daher eine Gesamtlänge von mindestens 1 Nucleotid bis höchstens 69 bis 72 Nucleotiden aufweisen. Wenn das längere Oligonucleotid eine Nucleotidsequenz umfasst, die gegenüber einer der in SEQ ID Nr. 8 bis 135 dargestellten Nucleotidsequenzen durch Addition von 1 bis 10 Nucleotiden mutiert ist, können die zusätzlichen Bereiche eine Gesamtlänge von mindestens 1 Nucleotid bis höchstens 68-71 Nucleotiden (im Falle der Addition eines Nucleotids) beziehungsweise 59-62 Nucleotiden (im Falle der Addition von 10 Nucleotiden) aufweisen. Die Gesamtlänge der zusätzlichen Bereiche des als längeres Oligonucleotid vorliegenden Nucleinsäuremoleküls beträgt insbesondere 1 bis etwa 60, vorzugsweise 1 bis etwa 50, besonders bevorzugt 1 bis etwa 40 und am bevorzugtesten 1 bis etwa 30 Nucleotide. Wenn das Nucleinsäuremolekül als Polynucleotid vorliegt, beträgt dessen Gesamtlänge mindestens 100 Nucleotide. Die Gesamtlänge der zusätzlichen Bereiche des Polynucleotids beträgt in Abhängigkeit davon, ob das erfindungsgemäße Polynucleotid eine der in SEQ ID Nr. 8 bis 135 dargestellten Sequenzen oder eine mutierte Sequenz davon umfasst, mindestens etwa 70 bis 80 Nucleotide bis etwa 1000 Nucleotide, vorzugsweise etwa 70-80 Nucleotide bis etwa 500 Nucleotide, besonders bevorzugt etwa 70-80 Nucleotide bis etwa 250 Nucleotide und am bevorzugtesten etwa 70-80 Nucleotide bis etwa 100 Nucleotide.

Der in dem Nucleinsäuremolekül enthaltene mindestens eine Bereich, der eine der in SEQ ID Nr. 8 bis 135 dargestellten Nucleotidsequenzen, insbesondere eine der in SEQ ID Nr. 1 bis 7, 19, 32, 41, 44, 48, 82 oder 117 bis 135 dargestellten Nucleotidsequenzen, eine mutierte Nucleotidsequenz davon oder eine komplementäre Nucleotidsequenz davon aufweist, kann am 5'-Ende und/oder am 3'-Ende der Sequenz des Nucleinsäuremoleküls und/oder zwischen den zusätzlichen Bereichen angeordnet sein.

In einer Ausführungsform der Offenbarung ist vorgesehen, dass diese zusätzlichen Bereiche des Nucleinsäuremoleküls Nucleotidsequenzen aufweisen, die zu Sequenzen eines Amplifikationsproduktes, das mittels eines Amplifikationsverfahrens unter Verwendung eines Nucleinsäure-Bereiches eines genitalen humanen Papillomavirus als Matrize und eines Primer-Paares erhältlich ist, komplementär sind. Beispielsweise können die zusätzlichen Bereiche mehrfache Wiederholungen des Bereiches, der eine der in SEQ ID Nr. 8 bis 135 dargestellten Nucleotidsequenzen, eine mutierte Nucleotidsequenz davon oder eine komplementäre Nucleotidsequenz davon aufweist, umfassen. Es ist möglich, dass die Wiederholungen dieses Bereiches jeweils durch Spacer mit einer Länge von mindestens einem Nucleotid oder mindestens einem Phosphat oder mindestens einem Kohlenstoffatom oder mindestens einer Aminogruppe getrennt sind.

In einer weiteren Ausführungsform der Offenbarung ist vorgesehen, dass die zusätzlichen Bereiche der Nucleinsäuremoleküle Nucleotidsequenzen aufweisen, die zu Sequenzen eines Amplifikationsproduktes, das mittels eines Amplifikationsverfahrens unter Verwendung eines Nucleinsäure-Bereiches eines genitalen humanen Papillomavirus als Matrize und eines Primer-Paares erhältlich ist, keine Komplementarität aufweisen und somit mit dem Amplifikationsprodukt nicht hybridisieren. Beispielsweise können die zusätzlichen Bereiche der Nucleinsäuremoleküle Poly-A- oder Poly-T-Nucleotidsequenzen aufweisen.

Die vorliegende Erfindung betrifft ebenfalls Nucleinsäuremoleküle, die eine Nucleotidsequenz aufweisen, die über ihre gesamte Länge hinweg zu der Nucleotidsequenz eines erfindungsgemäßen Nucleinsäuremoleküls komplementär ist. Erfindungsgemäß ist femer vorgesehen, dass die erfindungsgemäßen Nucleinsäuremoleküle als, vorzugsweise gereinigte und isolierte, DNA-Moleküle, RNA-Moleküle, PNA-Moleküle, LNA-Moleküle oder Mischformen davon vorliegen.

Die vorliegende Erfindung löst das ihr zugrunde liegende technische Problem auch durch die Bereitstellung eines Verfahrens zur Amplifikation eines Bereiches einer in einer biologischen Probe vorhandenen Nucleinsäure eines genitalen humanen Papillomavirus, umfassend die Durchführung eines Nucleinsäureamplifikations-Verfahrens unter Verwendung eines einen Forward-Primer und eine Reverse-Primer umfassenden Primer-Paares, wobei der Forward-Primer ausgewählt ist aus der Gruppe bestehend aus:
a) einem Oligonucleotid mit einer der in SEQ ID Nr. 1 bis 6 dargestellten Nucleotidsequenz,
b) einem Gemisch der Oligonucleotide nach a) und der Reverse-Primer ausgewählt ist aus der Gruppe bestehend aus:
   einem Oligonucleotid mit der in SEQ ID Nr. 7 dargestellten Nucleotidsequenz.

Unter Verwendung des erfindungsgemäßen Verfahrens können in vorteilhafter Weise spezifische Nucleinsäurebereiche des konservierten E1-Gens, einer Vielzahl genitaler humaner Papilloma-Viren amplifiziert werden. Die dabei erhaltenen Amplifikationsprodukte erlauben den Nachweis der Gegenwart eines Papilloma-Virus beziehungsweise mehrerer Papilloma-Viren in einer biologischen Probe, während das Fehlen eines Amplifikationsproduktes darauf hinweist, dass in der untersuchten Probe kein HPV-Virus vorhanden ist. Das unter Verwendung des erfindungsgemäßen Amplifikationsverfahrens erhaltene Amplifikationsprodukt kann dann mittels der offenbarten Oligonucleotid- und/oder Polynucleotid-Sonden zur Typisierung des nachgewiesenen Papilloma-Virus beziehungsweise der nachgewiesenen Papilloma-Viren eingesetzt werden.

Im Zusammenhang mit der vorliegenden Erfindung wird unter einer "biologischen Probe" ein Haut- oder Schleimhautabstrich, eine Organbiopsie, eine Gewebebiopsie, eine Körperflüssigkeit oder ein Körpersekret verstanden. Die Probe kann sowohl einem lebenden oder toten Organismus, Organ oder Gewebe entnommen sein. Eine "biologische Probe" kann erfindungsgemäß auch eine Kultur oder ein Kulturmedium sein, beispielsweise ein Medium, in dem menschliche oder tierische Zellen kultiviert wurden. Bei einer Probe im Sinne der Erfindung kann es sich auch um eine wässrige Lösung, Emulsion, Dispersion oder Suspension handeln, die isolierte und aufgereinigte humane Papillomaviren oder Bestandteile davon enthält. Eine biologische Probe kann bereits Aufreinigungsschritten unterworfen worden sein, kann aber auch ungereinigt vorliegen.

In einer bevorzugten Ausführungsform der Erfindung ist die biologische Probe ein Abstrich der Cervix uteri, eine frische Cervix uteri-Gewebeprobe, eine fixierte Cervix uteri-Gewebeprobe oder ein Schnittpräparat einer Cervix uteri-Gewebeprobe. Erfindungsgemäß ist vorgesehen, dass es sich bei der Probe insbesondere um eine solche Probe handelt, die im Rahmen einer zytologischen Untersuchung und/oder einer Kolposkopie zur Erfassung HPV-assoziierter Epithelveränderungen des subklinischen beziehungsweise klinisch-manifesten Stadiums einer HPV-Infektion entnommen wird.

Erfindungsgemäß ist vorgesehen, dass die zu amplifizierende Nucleinsäure vor der Amplifikation unter Verwendung der erfindungsgemäßen Primer aus der biologischen Probe aufgereinigt und/oder isoliert werden kann. Die Aufreinigung und Isolierung der zu amplifizierenden Nucleinsäure, d.h. der zu amplifizierenden HPV-DNA, kann unter Verwendung der im Stand der Technik bekannten Verfahren erfolgen.

In bevorzugter Ausführungsform der Erfindung ist vorgesehen, dass zur Nucleinsäureamplifikation ein PCR (Polymerase-KettenReaktion; polymerase chain reaction)-Verfahren eingesetzt wird. Bei dem PCR-Verfahren handelt es sich um ein Verfahren zur gezielten Vervielfältigung einer spezifischen Nucleinsäure oder eines Bereiches davon in einem Gemisch unterschiedlicher oder ähnlicher Sequenzen. Das Verfahren basiert darauf, dass bestimmte Reaktionsschritte, nämlich Denaturierung eines zu amplifizierenden doppelsträngigen Nucleinsäure-Moleküls, Anlagerung von Primern an die erhaltenen einzelsträngigen Nucleinsäure-Moleküle (annealing) und eine von den angelagerten Primern ausgehende Synthese von komplementären Strängen (extension), wobei die einzelsträngigen Nucleinsäure-Moleküle als Matrize dienen, mehrfach wiederholt werden. In einem ersten Schritt wird daher die zu amplifizierende doppelsträngige Nucleinsäure bei einer geeigneten Temperatur denaturiert, wobei als Matrizen dienende einzelsträngige Nucleinsäure erhalten werden. Danach erfolgt ein Absenken der Temperatur, wobei im Reaktionsansatz im Überschuss enthaltene Oligonucleotide (Primer), deren Sequenzen zum Anfang beziehungsweise zum Ende der zu amplifizierenden Nucleinsäuren komplementär sind, mit den komplementären Nucleinsäurebereichen hybridisieren. Bei einer Temperatur, die der Hybridisierungstemperatur von etwa 50°C bis 72°C entsprechen kann, die aber auch auf das Optimum von 72°C bis 75°C der, vorzugsweise hitzestabilen, DNA-Polymerase, eingestellt sein kann, synthetisiert die Polymerase dann ausgehend von den Primern Kopien der Ausgangs-Nucleinsäure in Form von Primer-Verlängerungsprodukten, wobei die Länge der Kopien durch den Abstand zwischen den Primern bestimmt wird. Eine Kettenreaktion wird dadurch erhalten, dass ein Zyklus durchgeführt wird, der die Denaturierung des Primer-Verlängerungsproduktes von der Matrize, die Behandlung der so erzeugten einsträngigen Moleküle mit den gleichen Primern und die Bildung weiterer Primer-Verlängerungsprodukte umfasst. Der Zyklus wird solange wiederholt, bis die zu amplifizierende Ziel-Nucleinsäure in einer gewünschten Menge vorliegt.

In bevorzugter Ausführungsform der Erfindung wird die PCR-Amplifikation der nachzuweisenden HPV-DNA unter folgenden Temperatur-Bedingungen durchgeführt:
a) Erhitzen auf 95°C, wobei die Temperatur pro Sekunde um 1°C erhöht wird,
b) Halten der Temperatur bei 95°C für 10 Minuten,
c) Durchführung von 40 Zyklen, jeweils umfassend 30 Sekunden bei 95°C, 30 Sekunden bei 55°C und 1 Minute bei 72°C,
d) Halten der Temperatur bei 72°C für 5 Minuten und
e) Abkühlen auf 4°C.

Erfindungsgemäß ist vorgesehen, dass die als Forward-Primer und Reverse-Primer eingesetzten Oligonucleotide in der Nucleinsäure-Amplifikationsreaktion jeweils in einer Konzentration von 0,5-1 pMol/µl eingesetzt werden. Besonders bevorzugt wird als Forward-Primer ein äquimolares Gemisch der Oligonucleotide mit den in SEQ ID Nr. 2 bis 6 dargestellten Nucleotidsequenzen eingesetzt, wobei jedes Oligonucleotid in einer Konzentration von 0,1-0,2 pMol/µl vorliegt, und als Reverse-Primer das Oligonucleotid mit der in SEQ ID Nr. 7 dargestellten Nucleotidsequenz in einer Konzentration von 0,5-1 pMol/µl. Die als Forward- und Reverse-Primer eingesetzten Oligonucleotide können als DNA-, RNA-, PNA- oder LNA-Moleküle oder Mischformen davon eingesetzt werden.

In einer anderen Ausführungsform der Erfindung ist vorgesehen, dass das Nucleinsäureamplifikations-Verfahren ein LCR (ligase chain reaction)-Verfahren, ein NASBA-Verfahren oder ein isothermisches Verfahren ist. Das LCR-Verfahren basiert auf der wiederholten Durchführung von zwei Reaktionsschritten, wobei im ersten Schritt eine doppelsträngige Nucleinsäure bei hoher Temperatur denaturiert wird. Im zweiten Schritt werden zwei Sätze benachbarter komplementärer Oligonucleotide an die im ersten Schritt erhaltene einzelsträngige Nucleinsäure hybridisiert und miteinander ligiert. Die Produkte der Ligierung aus dieser Reaktion dienen bei Wiederholung des Verfahrens als Matrizen. Wie die PCR resultiert auch das LCR-Verfahren in einer exponentiellen Amplifikation der Ligierungsprodukte.

Erfindungsgemäß ist insbesondere vorgesehen, dass im erfindungsgemäßen Amplifikationsverfahren ein spezifischer Bereich des HPV-Gens E1 amplifiziert wird. Erfindungsgemäß ist selbstverständlich vorgesehen, dass der amplifizierte Nucleinsäurebereich im Anschluss aufgereinigt und/oder isoliert werden kann, um beispielsweise eine HPV-Typisierung vorzunehmen.

Efindungsgemäß ist ebenfalls vorgesehen, dass das unter Verwendung des erfindungsgemäßen Verfahrens hergestellte Amplifikationsprodukt während oder nach der Amplifikation mit einer Markierung versehen werden kann, wodurch der spätere Nachweis des Amplifikationsproduktes, insbesondere unter Verwendung eines oder mehrerer der erfindungsgemäß als Sonden eingesetzten Oligonucleotide mit den in SEQ ID Nr. 8 bis 135 dargestellten Nucleotidsequenzen, vorzugsweise mit den in SEQ ID Nr. 1 bis 7, 19, 32, 41, 44, 48, 82 und 117 bis 135 dargestellten Nucleotidsequenzen, mutierten Nucleotidsequenzen davon oder komplementären Nucleotidsequenzen davon und/oder der erfindungsgemäßen-Nucleinsäuremoleküle, ermöglicht wird. Eine Markierung des Amplifikationsproduktes während der Amplifikationsreaktion kann beispielsweise unter Verwendung von markierten Primern und/oder markierten Nucleotiden erfolgen. Das Amplifikationsprodukt kann selbstverständlich auch nach Beendigung der Amplifikationsreaktion unter Verwendung von im Stand der Technik bekannten Verfahren erfolgen. Erfindungsgemäß kann das Amplifikationsprodukt zum Beispiel unter Verwendung von Fluoreszenz-Farbstoffen, Biotin, Haptenen, Antigenen, chemischen Gruppen, beispielsweise durch Verwendung aminoallyl-markierter Nucleotide oder durch Anbringen eines Markers mittels einer Succinimid-Reaktion mit dem aminoallylmarkierten Nucelotide, radioaktiven Stoffen, enzymatischen Markern etc. markiert werden. Die Detektion des markierten Amplifikationsproduktes kann beispielsweise unter Verwendung von Fluoreszenz-Verfahren, Chemolumineszenz-Verfahren, Densitometrie-Verfahren, Photometrie-Verfahren, Fällungsreaktionen, enzymatischen Reaktionen einschließlich enzymatischer Verstärkungsreaktionen, SPR ("surface plamon resonance")-Verfahren, Ellipsometrie-Verfahren, Messung des Brechungsindex, Messung der Reflektivität und ähnlichen Verfahren erfolgen.

Die vorliegende Offenbarung löst das ihr zugrunde liegende technische Problem auch durch die Bereitstellung eines Verfahrens zum Nachweis und/oder zur Bestimmung eines individuellen genitalen HPV-Genotyps, umfassend die Untersuchung einer in einer biologischen Probe vorhandenen Nucleinsäure eines genitalen humanen Papillomavirus, insbesondere die Untersuchung des HPV-Gens E1 oder eines Teils davon, durch Hybridisierung mit mindestens einer Sonde, wobei die Sonde ausgewählt ist aus der Gruppe bestehend aus:
a) HPV-Genotyp-spezifischen Oligonucleotiden mit den in SEQ ID Nr. 8 bis 135 dargestellten Nucleotidsequenzen, insbesondere den in SEQ ID Nr. 1 bis 7, 19, 32, 41, 44, 48, 82 oder 117 bis 135 dargestellten Nucleotidsequenzen,
b) Oligonucleotiden, die eine gegenüber einem der Oligonucleotide nach a) mutierte Nucleotidsequenz aufweisen,
c) Oligonucleotiden, die eine Nucleotidsequenz aufweisen, die gegenüber der eines Oligonucleotids nach a) oder b) komplementär ist
d) Nucleinsäuremolekülen, die mindestens einen Bereich umfassen, der eine der in SEQ ID Nr. 8 bis 135 dargestellten Nucleotidsequenzen, insbesondere eine der in SEQ ID Nr. 1 bis 7, 19, 32, 41, 44, 48, 82 oder 117 bis 135 dargestellten Nucleotidsequenzen, eine mutierte Nucleotidsequenz davon oder eine komplementäre Nucleotidsequenz davon aufweist, und
e) Gemischen der Oligonucleotide nach a) bis c) und/oder der Nucleinsäuremoleküle nach d),
und den Nachweis der Hybridisierung.

Das erfindungsgemäße Verfahren zum Nachweis und/oder zur Bestimmung eines individuellen genitalen HPV-Genotyps dient insbesondere zur Typisierung von HPV-Genotypen, deren Vorhandensein in einer biologischen Probe bereits mittels eines anderen Verfahrens gesichert nachgewiesen wurde. Der Nachweis des Vorliegens einer HPV-Infektion kann beispielsweise unter Verwendung des HCM-Verfahrens erfolgt sein, mit dessen Hilfe high risk-Typen von low risk-Typen unterschieden werden können.

In einer bevorzugten Ausführungsform des erfindungsgemäßen Verfahrens ist vorgesehen, dass die in der biologischen Probe vorhandene HPV-Nucleinsäure vor Hybridisierung mit der/den Oligonucleoitid- oder Nucleinsäuremolekül-Sonde(n) amplifiziert und ein Amplifikationsprodukt erhalten wurde, dass im erfindungsgemäßen Verfahren zur Typisierung mittels der erfindungsgemäßen Oligonucleotid- und/oder Nucleinsäuremolekül-Sonden untersucht werden kann. Die Amplifikation der HPV-Nucleinsäure erfolgt unter Verwendung des erfindungsgemäßen Verfahrens zur Amplifikation von HPV-Nucleinsäuren, das heißt unter Verwendung der Oligonucleotide mit den in SEQ ID Nr. 1, 2, 3, 4, 5 und 6 dargestellten Nucleotidsequenzen als Forward-Primer und des Oligonucleotids mit der in SEQ ID Nr. 7 dargestellten Nucleotidsequenz als Reverse-Primer. Erfindungsgemäß ist daher vorgesehen, dass die biologische Probe, die einer erfindungsgemäßen Typisierung unterworfen werden soll, einen amplifizierten Nucleinsäurebereich umfasst, der mittels des erfindungsgemäßen Verfahrens zur NucleinsäureAmplifikation erhalten wurde.

Selbstverständlich kann das offenbarte Verfahren zum Nachweis und/oder zur Bestimmung, d.h. zur Typisierung eines genitalen HPV-Genotyps auch an einer biologischen Probe durchgeführt werden, die keiner Amplifikation eines HPV-Nucleinsäurebereiches unterworfen wurde. In einer anderen Ausführungsform ist daher vorgesehen, dass die biologische Probe ein Abstrich der Cervix uteri, eine frische Cervix uteri-Gewebeprobe, eine fixierte Cervix uteri-Gewebeprobe oder ein Schnittpräparat einer solchen Gewebeprobe ist.

Die Hybridisierung mit den Oligonucleotid- oder Nucleinsäuremolekül-Sonden wird unter Bedingungen durchgeführt, die im Stand der Technik beschrieben sind.

Es ist vorgesehen, dass eine Hybridisierung mit einem Oligonucleotid mit der in SEQ ID Nr. 8, 9 oder 117 dargestellten Nucleotidsequenz, vorzugsweise der in SEQ ID Nr. 117 dargestellten Nucleotidsequenz, einer mutierten Nucleotidsequenz davon oder einer komplementären Nucleotidsequenz davon oder mit einem Nucleinsäuremolekül, das eine der in SEQ ID Nr: 8, 9 oder 117 dargestellten Nucleotidsequenzen, vorzugsweise die in SEQ ID Nr. 117 dargestellte Nucleotidsequenz, eine mutierte Nucleotidsequenz davon oder eine komplementäre Nucleotidsequenz davon umfasst, den HPV6-Genotyp, insbesondere HPV6b-Genotyp anzeigt.

Eine Hybridisierung mit einem Oligonucleotid mit der in SEQ ID Nr. 10, 11, 12, 13, 14, 15, 16, 17 oder 118 dargestellten Nucleotidsequenz, vorzugsweise der in SEQ ID Nr. 118 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 10, 11, 12, 13, 14, 15, 16, 17 oder 118 dargestellten Nucleotidsequenzen, vorzugsweise die in SEQ ID Nr. 118 dargestellte Nucleotidsequenz, eine mutierte Nucleotidsequenz davon oder eine komplementäre Nucleotidsequenz davon umfasst, zeigt den HPV11-Genotyp an.

Es ist vorgesehen, dass eine Hybridisierung mit einem Oligonucleotid mit der in SEQ ID Nr. 18, 19 oder 20, vorzugsweise der in SEQ ID Nr. 19 dargestellten Nucleotidsequenz,dargestellten Nucleotidsequenz, einer mutierten Nucleotidsequenz davon oder einer komplementären Nucleotidsequenz davon oder mit einem Nucleinsäuremolekül, das eine der in SEQ ID Nr. 18, 19 oder 20 dargestellten Nucleotidsequenzen, vorzugsweise die in SEQ ID Nr. 19 dargestellte Nucleotidsequenz, eine mutierte Nucleotidsequenz davon oder eine komplementäre Nucleotidsequenz davon umfasst, den HPV16-Genotyp anzeigt.

Es ist vorgesehen, dass eine Hybridisierung mit einem Oligonucleotid mit der in SEQ ID Nr. 21, 22, 23, 24 oder 119 dargestellten Nucleotidsequenz, vorzugsweise der in SEQ ID Nr. 119 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 21, 22, 23, 24 oder 119 dargestellten Nucleotidsequenzen, vorzugsweise die in SEQ ID Nr. 119 dargestellte Nucleotidsequenz, eine mutierte Nucleotidsequenz davon oder eine komplementäre Nucleotidsequenz davon umfasst, den HPV18-Genotyp anzeigt.

Es ist darüber hinaus vorgesehen, dass eine Hybridisierung mit einem Oligonucleotid mit der in SEQ ID Nr. 25, 26, 27 oder 28 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 25, 26, 27 oder 28 dargestellten Nucleotidsequenzen, eine mutierte Nucleotidsequenz davon oder eine komplementäre Nucleotidsequenz davon umfasst, den HPV26-Genotyp anzeigt.

Eine Hybridisierung mit einem Oligonucleotid mit der in SEQ ID Nr. 29, 30, 31 oder 120 dargestellten Nucleotidsequenz, vorzugsweise der in SEQ ID Nr. 120 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 29, 30, 31 oder 120 dargestellten Nucleotidsequenzen, vorzugsweise die in SEQ ID Nr. 120 dargestellte Nucleotidsequenz, eine mutierte Nucleotidsequenz davon oder eine komplementäre Nucleotidsequenz davon umfasst, zeigt den HPV31-Genotyp an.

Es ist darüber hinaus vorgesehen, dass eine Hybridisierung mit einem Oligonucleotid mit der in SEQ ID Nr. 32, 33 oder 34 dargestellten Nucleotidsequenz, vorzugsweise der in SEQ ID Nr. 32 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 32, 33 oder 34 dargestellten Nucleotidsequenzen, vorzugsweise die in SEQ ID Nr. 32 dargestellte Nucleotidsequenz, eine mutierte Nucleotidsequenz davon oder eine komplementäre Nucleotidsequenz davon umfasst, den HPV33-Genotyp anzeigt.

In einer weiteren Ausführungsform ist vorgesehen, dass eine Hybridisierung mit einem Oligonucleotid mit der in SEQ ID Nr. 35, 36, 37, 38 oder 39 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 35, 36, 37, 38 oder 39 dargestellten Nucleotidsequenzen, eine mutierte Nucleotidsequenz davon oder eine komplementäre Nucleotidsequenz davon umfasst, den HPV34-Genotyp anzeigt.

Es ist vorgesehen, dass eine Hybridisierung mit einem Oligonucleotid mit der in SEQ ID Nr. 40 oder 41 dargestellten Nucleotidsequenz, vorzugsweise der in SEQ ID Nr. 41 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 40 oder 41 dargestellten Nucleotidsequenzen, vorzugsweise die in SEQ ID Nr. 41 dargestellte Nucleotidsequenz, eine mutierte Nucleotidsequenz davon oder eine komplementäre Nucleotidsequenz davon umfasst, den HPV35-Genotyp, insbesondere HPV35h-Genotyp anzeigt.

Es ist ferner vorgesehen, dass eine Hybridisierung mit einem Oligonucleotid mit der in SEQ ID Nr. 42, 43, 44, 45 oder 46 dargestellten Nucleotidsequenz, vorzugsweise der in SEQ ID Nr. 44 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 42, 43, 44, 45 oder 46 dargestellten Nucleotidsequenzen, vorzugsweise die in SEQ ID Nr. 44 dargestellte Nucleotidsequenz, eine mutierte Nucleotidsequenz davon oder eine komplementäre Nucleotidsequenz davon umfasst, den HPV39-Genotyp anzeigt.

Eine Hybridisierung mit einem Oligonucleotid mit der in SEQ ID Nr. 47, 48, 49 oder 50 dargestellten Nucleotidsequenz, vorzugsweise der in SEQ ID Nr. 48 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 47, 48, 49 oder 50 dargestellten Nucleotidsequenzen, vorzugsweise die in SEQ ID Nr. 48 dargestellte Nucleotidsequenz, eine mutierte Nucleotidsequenz davon oder eine komplementäre Nucleotidsequenz davon umfasst, zeigt den HPV40-Genotyp an.

Es ist darüber hinaus vorgesehen, dass eine Hybridisierung mit einem Oligonucleotid mit der in SEQ ID Nr. 51, 52 oder 121 dargestellten Nucleotidsequenz, vorzugsweise der in SEQ ID Nr. 121 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 51, 52 oder 121 dargestellten Nucleotidsequenzen, vorzugsweise die in SEQ ID Nr. 121 dargestellte Nucleotidsequenz, eine mutierte Nucleotidsequenz davon oder eine komplementäre Nucleotidsequenz davon umfasst, den HPV42-Genotyp anzeigt.

Es ist darüber hinaus vorgesehen, dass eine Hybridisierung mit einem Oligonucleotid mit der in SEQ ID Nr. 122 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 122 dargestellten Nucleotidsequenzen, eine mutierte Nucleotidsequenz davon oder eine komplementäre Nucleotidsequenz davon umfasst, den HPV43-Genotyp anzeigt.

Eine Hybridsierung mit einem Oligonucleotid mit der in SEQ ID Nr. 53, 54, 55 oder 123 dargestellten Nucleotidsequenz, vorzugsweise der in SEQ ID Nr. 123 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 53, 54, 55 oder 123 dargestellten Nucleotidsequenzen, vorzugsweise die in SEQ ID Nr. 123 dargestellte Nucleotidsequenz, eine mutierte Nucleotidsequenz davon oder eine komplementäre Nucleotidsequenz davon umfasst, zeigt den HPV44-Genotyp an.

Es ist ferner vorgesehen, dass eine Hybridisierung mit einem Oligonucleotid mit der in SEQ ID Nr. 56, 57, 58, 59, 60, 61 oder 124 dargestellten Nucleotidsequenz, vorzugsweise der in SEQ ID Nr. 124 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 56, 57, 58, 59, 60, 61 oder 124 dargestellten Nucleotidsequenzen, vorzugsweise die in SEQ ID Nr. 124 dargestellte Nucleotidsequenz, eine mutierte Nucleotidsequenz davon oder eine komplementäre Nucleotidsequenz davon umfasst, den HPV45-Genotyp anzeigt.

Eine Hybridisierung mit einem Oligonucleotid mit der in SEQ ID Nr. 62, 63, 64 oder 125 dargestellten Nucleotidsequenz, vorzugsweise der in SEQ ID Nr. 125 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 62, 63, 64 oder 125 dargestellten Nucleotidsequenzen, vorzugsweise die in SEQ ID Nr. 125 dargestellte Nucleotidsequenz, eine mutierte Nucleotidsequenz davon oder eine komplementäre Nucleotidsequenz davon umfasst, zeigt den HPV51-Genotyp an.

Es ist ferner vorgesehen, dass eine Hybridisierung mit einem Oligonucleotid mit der in SEQ ID Nr. 65, 66, 67 oder 126 dargestellten Nucleotidsequenz, vorzugsweise der in SEQ ID Nr. 126 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 65, 66, 67 oder 126 dargestellten Nucleotidsequenzen, vorzugsweise die in SEQ ID Nr. 126 dargestellte Nucleotidsequenz, eine mutierte Nucleotidsequenz davon oder eine komplementäre Nucleotidsequenz davon umfasst, den HPV52-Genotyp anzeigt.

Eine Hybridisierung mit einem Oligonucleotid mit der in SEQ ID Nr. 68, 69, 70, 71, 72, 73 oder 127 dargestellten Nucleotidsequenz, vorzugsweise der in SEQ ID Nr. 127 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 68, 69, 70, 71, 72, 73 oder 127 dargestellten Nucleotidsequenzen, vorzugsweise die in SEQ ID Nr. 127 dargestellte Nucleotidsequenz, eine mutierte Nucleotidsequenz davon oder eine komplementäre Nucleotidsequenz davon umfasst, zeigt den HPV53-Genotyp an.

In einer weiteren Ausführungsform des Verfahrens ist vorgesehen, dass eine Hybridisierung mit einem Oligonucleotid mit der in SEQ ID Nr. 74, 75, 76 oder 77 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 74, 75, 76 oder 77 dargestellten Nucleotidsequenzen, eine mutierte Nucleotidsequenz davon oder eine komplementäre Nucleotidsequenz davon umfasst, den HPV54-Genotyp anzeigt.

Eine Hybridisierung mit einem Oligonucleotid mit der in SEQ ID Nr. 78, 79, 80, 81 oder 128 dargestellten Nucleotidsequenz, vorzugsweise der in SEQ ID Nr. 128 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 78, 79, 80, 81 oder 128 dargestellten Nucleotidsequenzen, vorzugsweise die in SEQ ID Nr. 128 dargestellte Nucleotidsequenz, eine mutierte Nucleotidsequenz davon oder eine komplementäre Nucleotidsequenz davon umfasst, zeigt den HPV56-Genotyp an.

Es ist ferner vorgesehen, dass eine Hybridisierung mit einem Oligonucleotid mit der in SEQ ID Nr. 82, 83, 84, 85 oder 86 dargestellten Nucleotidsequenz, vorzugsweise der in SEQ ID Nr. 82 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 82, 83, 84, 85 oder 86 dargestellten Nucleotidsequenzen, vorzugsweise die in SEQ ID Nr. 82 dargestellte Nucleotidsequenz, eine mutierte Nucleotidsequenz davon oder eine komplementäre Nucleotidsequenz davon umfasst, den HPV58-Genotyp anzeigt.

Eine Hybridisierung mit einem Oligonucleotid mit der in SEQ ID Nr. 87 oder 129 dargestellten Nucleotidsequenz, vorzugsweise der in SEQ ID Nr. 129 dargestellten Nucleotidsequenz, einer mutierten Nucleotidsequenz davon oder einer komplementären Nucleotidsequenz davon oder mit einem Nucleinsäuremolekül, das die in SEQ ID Nr. 87 oder 129 dargestellte Nucleotidsequenz, vorzugsweise die in SEQ ID Nr. 129 dargestellten Nucleotidsequenz, eine mutierte Nucleotidsequenz davon oder eine komplementäre Nucleotidsequenz davon umfasst, zeigt den HPV59-Genotyp an.

In einer weiteren Ausführungsform ist vorgesehen, dass eine Hybridisierung mit einem Oligonucleotid mit der in SEQ ID Nr. 88, 89, 90, 91, 92, 93 oder 94 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 88, 89, 90, 91, 92, 93 oder 94 dargestellten Nucleotidsequenzen, eine mutierte Nucleotidsequenz davon oder eine komplementäre Nucleotidsequenz davon umfasst, den HPV61-Genotyp anzeigt.

Eine Hybridisierung mit einem Oligonucleotid mit der in SEQ ID Nr. 95, 96, 97 oder 130 dargestellten Nucleotidsequenz, vorzugsweise der in SEQ ID Nr. 130 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 95, 96, 97 oder 130 dargestellten Nucleotidsequenzen, vorzugsweise die in SEQ ID Nr. 130 dargestellte Nucleotidsequenz, eine mutierte Nucleotidsequenz davon oder eine komplementäre Nucleotidsequenz davon umfasst, zeigt den HPV66-Genotyp an.

Es ist darüber hinaus vorgesehen, dass eine Hybridisierung mit einem Oligonucleotid mit der in SEQ ID Nr. 98, 99, 100, 101 oder 102 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 98, 99, 100, 101 oder 102 dargestellten Nucleotidsequenzen, eine mutierte Nucleotidsequenz davon oder eine komplementäre Nucleotidsequenz davon umfasst, den HPV67-Genotyp anzeigt.

Eine Hybridisierung mit einem Oligonucleotid mit der in SEQ ID Nr. 103, 104, 105, 131 oder 132 dargestellten Nucleotidsequenz, vorzugsweise der in SEQ ID Nr. 131 oder 132 dargestellten Nucleotidsequenz,einer mutierten Nucleotidsequenz davon oder einer komplementären Nucleotidsequenz davon oder mit einem Nucleinsäuremolekül, das eine der in SEQ ID Nr. 103, 104, 105, 131 oder 132 dargestellten Nucleotidsequenzen, vorzugsweise die in SEQ ID Nr. 131 oder 132 dargestellte Nucleotidsequenz, eine mutierte Nucleotidsequenz davon oder eine komplementäre Nucleotidsequenz davon umfasst, zeigt den HPV68-Genotyp an.

In einer weiteren Ausführungsform ist vorgesehen, dass eine Hybridisierung mit einem Oligonucleotid mit der in SEQ ID Nr. 106, 107, 108 oder 133 dargestellten Nucleotidsequenz, vorzugsweise der in SEQ ID Nr. 133 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 106, 107, 108 oder 133 dargestellten Nucleotidsequenzen, vorzugsweise die in SEQ ID Nr. 133 dargestellte Nucleotidsequenz, eine mutierte Nucleotidsequenz davon oder eine komplementäre Nucleotidsequenz davon umfasst, den HPV70-Genotyp anzeigt.

Eine Hybridisierung mit einem Oligonucleotid mit der in SEQ ID Nr. 109, 110, 111, 112 oder 134 dargestellten Nucleotidsequenz, vorzugsweise der in SEQ ID Nr. 134 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 109, 110, 111, 112 oder 134 dargestellten Nucleotidsequenzen, vorzugsweise die in SEQ ID Nr. 134 dargestellte Nucleotidsequenz, eine mutierte Nucleotidsequenz davon oder eine komplementäre Nucleotidsequenz davon umfasst, zeigt den HPV73-Genotyp an.

Eine Hybridisierung mit einem Oligonucleotid mit der in SEQ ID Nr. 113, 114, 115, 116 oder 135 dargestellten Nucleotidsequenz, vorzugsweise der in SEQ ID Nr. 135 dargestellten Nucleotidsequenz, einer mutierten Nucleotidsequenz davon oder einer komplementären Nucleotidsequenz davon oder mit einem Nucleinsäuremolekül, das eine der in SEQ ID Nr. 113, 114, 115, 116 oder 135 dargestellten Nucleotidsequenzen, vorzugsweise die in SEQ ID Nr. 135 dargestellte Nucleotidsequenz, eine mutierte Nucleotidsequenz davon oder eine komplementäre Nucleotidsequenz davon umfasst, zeigt den HPV82-Genotyp an.

Das als Sonde eingesetzte Oligonucleotid oder Nucleinsäuremolekül kann als DNA-, RNA-, PNA- oder LNA-Molekül oder Mischform davon vorliegen.

Die erfindungsgemäßen Verfahren zur Amplifikation von Nucleinsäurebereichen humaner Papillomaviren und zum Nachweis und/oder zur Bestimmung genitaler HPV-Genotypen können insbesondere zur Diagnose und/oder zur Früherkennung von Erkrankungen, die von genitalen humanen Papillomaviren verursacht werden, eingesetzt werden. Die erfindungsgemäßen Verfahren können in bevorzugter Ausführungsform als Teil eines Verfahrens zur Diagnose und/oder Früherkennung von Krankheiten, insbesondere Krebserkrankungen, beispielsweise Gebärmutterhalskrebs, eingesetzt werden. Die erfindungsgemäßen Verfahren können ebenfalls im Rahmen einer Früherkennungs-Untersuchung von Krebserkrankungen eingesetzt werden. Die erfindungsgemäße Verfahren führen zu gesicherten positiven Befunden hinsichtlich einer HPV-Infektion und können somit als Ausgangspunkt für eine zielgerichtete Therapie dienen.

Die vorliegende Offenbarung löst das ihr zugrunde liegende technische Problem auch durch die Bereitstellung eines Nucleotid-Arrays zum Nachweis und/oder zur Bestimmung des Genotyps eines in einer biologischen Probe enthaltenen humanen Papillomavirus, insbesondere unter Verwendung des erfindungsgemäßen Verfahrens zum Nachweis und/oder zur Bestimmung des Genotyps von HPV-Viren, umfassend einen festen Träger mit einer Oberfläche und mindestens ein an die Träger-Oberfläche gebundenes erstes Oligonucleotid oder Nucleinsäuremolekül, das zum Nachweis und/oder zur Bestimmung eines genitalen HPV-Genotyps geeignet ist, ausgewählt aus der Gruppe bestehend aus:
a) HPV-Genotyp-spezifischen Oligonucleotiden mit den in SEQ ID Nr. 8 bis 135 dargestellten Nucleotidsequenzen, vorzugsweise den in SEQ ID Nr. 19, 32, 41, 44, 48, 82 und 117 bis 135 dargestellten Nucleotidsequenzen,
b) Oligonucleotiden, die eine gegenüber einem der Oligonucleotide nach a) mutierte Nucleotidsequenz aufweisen,
c) Oligonucleotiden, die eine Nucleotidsequenz aufweisen, die gegenüber der eines Oligonucleotids nach a) oder b) komplementär ist,
d) Nucleinsäuremolekülen, die einen Bereich umfassen, der eine der in SEQ ID Nr. 8 bis 135 dargestellten Nucleotidsequenzen, vorzugsweise eine der in SEQ ID Nr. 19, 32, 41, 44, 48, 82 und 117 bis 135 dargestellten Nucleotidsequenzen, eine mutierte Nucleotidsequenz davon oder eine komplementäre Nucleotidsequenz davon aufweist, und
e) Gemischen der Oligonucleotide nach a) bis c) und/oder der Nucleinsäuremoleküle nach d).

Im Zusammenhang mit der vorliegenden Erfindung wird unter einem "Nucleotid-Array" oder einem "Nucleotid-Chip" eine Vorrichtung verstanden, die eine Vielzahl verschiedener Nucleinsäuremoleküle oder Nucleotidsequenzen, beispielsweise DNA-Moleküle, RNA-Moleküle, PNA-Moleküle, LNA-Moleküle und/oder Mischformen davon, in immobilisierter Form enthält und mit deren Hilfe mittels Nucleinsäure-Hybridisierung eine kleine Menge einer komplementären Nucleinsäure in einer kleinen Probenflüssigkeit nachgewiesen werden kann. Unter Verwendung der offenbarten Nucleotid-Arrays ist einerseits der einfache Nachweis von humanen Papillomaviren in einer biologischen Probe möglich, andererseits kann aber auch eine Unterscheidung zwischen genitalen HPV-Genotypen, insbesondere eine Unterscheidung zwischen Hoch-Risiko- und Niedrig-Risiko-Genotypen sowie eine Typisierung individueller genitaler HPV-Genotypen vorgenommen werden.

Unter dem "Träger" des Nucleotid-Arrays wird im Zusammenhang mit der vorliegenden Erfindung eine Vorrichtung verstanden, die eine Trägerplatte mit einer Oberfläche umfasst, an der ein biologisch aktives Molekül, beispielsweise eine Nucleinsäure, immobilisiert oder fixiert werden kann. Der Träger des Nucleotid-Arrays wird also zur Herstellung eines Nucleotid-Arrays verwendet, indem biologisch aktive Moleküle, insbesondere Nucleinsäuren oder Teile davon an der Oberfläche des Trägers immobilisiert oder fixiert werden. Die biologisch aktiven Moleküle sind dabei in Form von Spots auf der Trägeroberfläche angeordnet.

Unter der "Trägerplatte" des Nucleotid-Array-Trägers wird ein dünnes und ebenes Element mit beispielsweise rechteckiger Form verstanden, das insbesondere aus einem Metall, einem Metalloxid, einem Kunststoff, einer Membran, Glas, Keramik, Gel etc. oder einem Hybrid beziehungsweise einer Kombination dieser Materialien besteht. Im Zusammenhang mit der vorliegenden Erfindung bedeutet dies, dass die Trägerplatte des Trägers beispielsweise vollständig aus einem der vorstehend als Beispiel genannten Materialien besteht oder diese im Wesentlichen enthält oder vollständig aus einer Kombination dieser Materialien besteht oder diese im Wesentlichen enthält. Die Trägerplatte oder ihre Oberfläche besteht dabei zumindest zu etwa 50 %, 60 %, vorzugsweise zu etwa 70 %, bevorzugt zu etwa 80 % und am bevorzugtesten zu etwa 100 % aus einem der vorstehend als Beispiel genannten Materialien oder einer Kombination solcher Materialien. In bevorzugter Ausführungsform besteht die Trägerplatte des erfindungsgemäßen Nucleotid-Arrays zu etwa 100 % aus Kunststoff.

Die Oberfläche des Trägers umfasst vorzugsweise ein Material, das nicht glatt ist, sondern rau, und/oder das nicht impermeabel ist, sondern permeabel, oder besteht daraus oder enthält dieses. In einer bevorzugten Ausführungsform besteht die Oberfläche des Trägers aus einem anderen Material als die Trägerplatte. Beispielsweise kann die Oberfläche aus einem Material wie Nitrocellulose bestehen oder dieses enthalten, während der Träger selbst aus einem Kunststoff, Glas, Keramik oder einem Metall besteht. In einer weiteren bevorzugten Ausführungsform kann die Oberfläche des Trägers auch aus dem zur Herstellung der Trägerplatte verwendeten Material bestehen. Vorzugsweise weist dieses Material eine raue und/oder permeable Oberfläche auf.

In einer bevorzugten Ausführungsform ist der Träger des erfindungsgemäßen Nucleotid-Arrays plättchenförmig, beispielsweise in Form eines Objektträgers, oder plättchenförmig mit Vertiefungen, beispielsweise als Chamberslide oder als Mikrotiterplatte mit Abmessungen gemäß Empfehlung der SBS (Society of Biomolecular Screening) ausgebildet.

Es sind die ersten Oligonucleotide oder Nucleinsäuremoleküle, insbesondere die Oligonucleotide mit den in SEQ ID Nr. 8 bis 135 dargestellten Nucleotidsequenzen, vorzugsweise die Oligonucleotide mit den in SEQ ID Nr. 19, 32, 41, 44, 48, 82 und 117 bis 135 dargestellten Nucleotidsequenzen, auf der Oberfläche des Trägers innerhalb eines definierten Analysebereiches in Form von Spots angeordnet. Es ist ferner vorgesehen, dass die Oberfläche des Trägers neben dem Analysebereich mindestens einen Kontrollbereich aufweist. Der Analysebereich und der Kontrollbereich können dabei auf der Oberfläche des Trägers an jeder beliebigen, definierten Stelle angeordnet sein.

Der auf der Trägeroberfläche angeordnete Kontrollbereich umfasst eine Kontrolle zur Orientierung des Trägers, eine Amplifikations-Kontrolle, eine Hybridisierungs-Kontrolle, eine Proben-Kontrolle und/oder eine Print-Kontrolle.

In einer Ausführungsform ist vorgesehen, dass die Kontrolle zur Orientierung des Trägers mindestens ein zweites Oligonucleotid oder Nucleinsäuremolekül umfasst. Das zweite Oligonucleotid oder Nucleinsäuremolekül ist vorzugsweise markiert, vorzugsweise fluoreszenzmarkiert. Das zweite Oligonucleotid oder Nucleinsäuremolekül wird vorzugsweise in mindestens drei Spots so auf der Träger-Oberfläche angeordnet, dass eine Orientierung des Nucleotid-Arrays möglich ist.

In einer weiteren Ausführungsform ist vorgesehen, dass die Amplifikationskontrolle mindestens ein drittes Oligonucleotid oder Nucleinsäuremolekül umfasst. Vorzugsweise ist das dritte Oligonucleotid oder Nucleinsäuremolekül als Sonde zum Nachweis eines Amplifikationsproduktes geeignet, wobei das Amplifikationsprodukt mittels eines Amplifikationsverfahrens unter Verwendung einer Kontroll-Nucleinsäure als Matrize und eines Primer-Paares, d.h. eines Oligonucleotides mit einer der in SEQ ID Nr. 1 bis 6 dargestellten Nucleotidsequenzen als Forward-Primer und eines Oligonucleotids mit der in SEQ ID Nr. 7 dargestellten Nucleotidsequenz als Reverse-Primer, erhältlich ist. In besonders bevorzugter Ausführungsform ist vorgesehen, dass die Kontroll-Nucleinsäure vorzugsweise eine Länge und einen GC-Gehalt aufweist, der der Länge und dem GC-Gehalt des Amplifikationsproduktes entspricht, das mittels des erfindungsgemäßen Amplifikationsverfahrens unter Verwendung des E1-Gens, eines genitalen humanen Papillomavirus als Matrize, eines Oligonucleotids mit einer der in SEQ ID Nr. 1 bis 6 dargestellten Nucleotidsequenzen als Forward-Primer und eines Oligonucleotids mit der in SEQ ID Nr. 7 dargestellten Nucleotidsequenz als Reverse-Primer erhältlich ist.

In einer weiteren bevorzugten Ausführungsform ist vorgesehen, dass die Hybridisierungskontrolle mindestens ein viertes Oligonucleotid oder Nucleinsäuremolekül umfasst. Vorzugsweise sind mindestens zwei bis zehn Spots des vierten Oligonucleotids oder Nucleinsäuremoleküls auf der Träger-Oberfläche angeordnet. Erfindungsgemäß ist vorgesehen, dass die einzelnen Spots der Hybridisierungskontrolle unterschiedliche definierte Mengen des vierten Oligonucleotids oder Nucleinsäuremoleküls aufweisen. Vorzugsweise umfasst die Hybridisierungskontrolle Spots mit einer Verdünnungsreihe des vierten Oligonucleotids oder Nucleinsäuremoleküls.

In einer weiteren bevorzugten Ausführungsform ist vorgesehen, dass die Proben-Kontrolle mindestens ein fünftes Oligonucleotid oder Nucleinsäuremolekül umfasst. Das fünfte Oligonucleotid oder Nucleinsäuremolekül ist vorzugsweise als Sonde zum Nachweis eines in allen Zellen eines menschlichen Organismus vorkommenden Gens geeignet. In bevorzugter Ausführungsform handelt es sich bei dem unter Verwendung des fünften Oligonucleotids oder Nucleinsäuremoleküls nachzuweisenden Gen um das humane ADAT1- (t-RNA-spezifische Adenosindeaminase. 1; Gene Bank Accession: NM_012091)-Gen.

In einer weiteren bevorzugten Ausführungsform ist vorgesehen, dass die Print-Kontrolle mindestens ein sechstes Oligonucleotid oder Nucleinsäuremolekül umfasst. Das sechste Oligonucleotid oder Nucleinsäuremolekül kann in Form separater Spots auf der Träger-Oberfläche angeordnet sein. Besonders bevorzugt ist das als Print-Kontrolle eingesetzte sechste Oligonucleotid oder Nucleinsäuremolekül jedoch in allen auf dem Nucleotid-Array-Träger angeordneten Spots mit Ausnahme der Spots des als Hybridisierungskontrolle dienenden vierten Oligonucleotids/Nucleinsäuremoleküls enthalten. Das heißt, in dieser Ausgestaltung umfasst jeder Spot des zum HPV-Genotyp-Nachweis dienenden ersten Oligonucleotids/Nucleinsäuremoleküls, jeder Spot des als Kontrolle zur Träger-Orientierung dienenden zweiten Oligonucleotids/Nucleinsäuremoleküls, jeder Spot des als Amplifikationskontrolle dienenden dritten Oligonucleotids/Nucleinsäuremoleküls und jeder Spot des als Proben-Kontrolle dienenden fünften Oligonucleotids/Nucleinsäuremoleküls auch das als Print-Kontrolle dienende sechste Oligonucleotid/Nucleinsäuremolekül. Vorzugsweise wird das sechste Oligonucleotid oder Nucleinsäuremolekül gemeinsam mit dem ersten, zweiten, dritten beziehungsweise fünften Oligonucleotid oder Nucleinsäuremolekül in einem Print-Schritt als Spot auf den Träger des Nucleotid-Arrays aufgebracht.

Die auf dem Nucleotid-Array fixierten Oligonucleotide oder Nucleinsäuremoleküle können erfindungsgemäß sowohl als DNA-Moleküle, RNA-Moleküle, PNA-Moleküle, LNA-Moleküle oder Mischformen davon ausgebildet sein.

In einer weiteren Ausführungsform ist vorgesehen, dass die auf dem Träger des Nucleotid-Arrays aufgebrachten ersten, dritten, vierten, fünften und sechsten Oligonucleotide oder Nucleinsäuremoleküle keine Markierung aufweisen, während das als Orientierungskontrolle dienende zweite Oligonucleotid oder Nucleinsäuremolekül eine Markierung, vorzugsweise eine Fluoreszenz-Markierung aufweist.

In einer weiteren bevorzugten Ausführungsform ist vorgesehen, dass der Nucleotid-Array einen Dot-Code aufweist, anhand dessen es möglich ist, den erfindungsgemäßen Nucleotid-Array eindeutig zu identifizieren und von anderen Nucleotid-Chips, die beispielsweise die gleiche Form und/oder die gleiche oder eine ähnliche Anordnung von Spots wie der erfindungsgemäße Nucleotid-Array aufweisen, jedoch für andere Zwecke eingesetzt werden und daher andere fixierte Sonden aufweisen, zu unterscheiden. Der erfindungsgemäße Dot-Code sichert darüber hinaus auch, dass bei der Software-gestützten Auswertung der unter Verwendung des erfindungsgemäßen Nucleotid-Arrays erhaltenen Ergebnisse die korrekte Software zur Analyse eingesetzt wird. Da die relevanten Informationen bezüglich des Nucleotid-Arrays direkt auf dem Nucleotid-Chip platziert werden, ist eine zusätzliche Markierung des Chips nicht mehr erforderlich. Der Chip kann dennoch eine zusätzliche Markierung aufweisen. Falls diese Markierung während der Verwendung des Nucleotid-Arrays verloren geht, sichert der erfindungsgemäße Dot-Code die eindeutige Identifizierbarkeit des Chips. Erfindungsgemäß ist vorgesehen, dass der Dot-Code während der Chip-Herstellung auf diesen aufgebracht wird. Der erfindungsgemäße Dot-Code kann selbstverständlich nicht nur für den erfindungsgemäßen Nucleotid-Array, der zum Nachweis und/oder zur Bestimmung des Genotyps eines humanen Papillomavirus dient, eingesetzt werden, sondern auch für andere Nucleotid-Chips, die beispielsweise zur Bestimmung oder zum Nachweis eines spezifischen Gens oder Genproduktes, zum Nachweis spezifischer Bakterien etc. dienen.

Es vorgesehen, dass der erfindungsgemäße Dot-Code mindestens Informationen bezüglich des vorgesehenen Anwendungstyps eines Nucleotid-Arrays, beispielsweise Nachweis und/oder Bestimmung des Genotyps humaner Papillomaviren, der Batch-Nummer und der Chip-Nummer enthält. Selbstverständlich kann der erfindungsgemäße Dot-Code noch weitere Informationen enthalten.

Es ist vorgesehen, dass der Anwendungstyp mittels einer unikalen Nummer gekennzeichnet wird. Beispielsweise kann der erfindungsgemäße Nucleotid-Array, der zum Nachweis und/oder Bestimmung des Genotyps humaner Papillomaviren eingesetzt wird, durch das Zeichen "#1" gekennzeichnet wird. Ein Nucleotid-Chip, der zur Bestimmung und/oder zum Nachweis einer spezifischen Bakterien-Art eingesetzt werden soll, kann beispielsweise durch das Zeichen "#2" gekennzeichnet werden. Dieses Zeichen kann mittels eines beliebigen numerischen Codes codiert werden, beispielsweise mittels eines binären Systems, eines Dezimalsystems, etc.. Bei dem 1-of-n-Codierungsverfahren gibt es n Positionen, wobei n eine ganze Zahl ist und eine der n Positionen positiv ist. Bei dem n-ary-Codierungsverfahren gibt es m Positionen, die n Intensitätsstufen aufweisen können. Bei binärer Codierung bedeutet dies, dass es zwei verschiedene Intensitätsstufen gibt, so dass es m Positionen gibt, die entweder "1" (positives Signal) oder "0" (negatives Signal) sein können. Bei Dezimalcodierung können 2 Positionen 100 verschiedene Nummern codieren, wobei jede Position 10 verschiedene Intensitätsstufen aufweisen kann.

Die Codierung kann auch einen zweidimensionalen Array von Spots umfassen, wobei eine binäre Codierung in zwei Dimensionen entsprechend 2^(n*m) erreicht wird, wobei n und m die Dimensionen des Arrays darstellen. Das Lesen (read out) des Dot-Codes kann binär oder analog erfolgen. Bei einem binären Readout wird einem bestimmten Spot entweder ein "Vorhanden" oder ein "Nicht-Vorhanden" zugewiesen. Im Gegensatz zu dem binären readout wird beim analogen Readout jedem Spot ein numerischer Wert zugewiesen. Dieser numerische Wert enthält selbst Informationen und kann beispielsweise die Größe des Spots oder die Signalintensität des Spots betreffen.

Zusätzliche Informationen auf dem Nucleotid-Array können darüber hinaus durch die Verwendung und/oder Kombination unterschiedlicher Mengen von zwei oder mehr Farben auf dem Nucleotid-Array erhalten werden, wobei die zwei oder mehr Farben in einem oder mehr Spots enthalten sein können.

Es ist bei Verwendung des Nucleotid-Arrays zum Nachweis und/oder zur Bestimmung des Genotyps eines humanen Papillomavirus, wobei insbesondere das Amplifikationsprodukt eingesetzt wird, das unter Verwendung des erfindungsgemäßen Verfahrens zur Amplifikation eines Bereiches eines genitalen humanen Papillomavirus erhalten wird, vorgesehen, dass das zu analysierende Amplifikationsprodukt in markierter Form eingesetzt wird. Das eingesetzte Amplifikationsprodukt kann bereits während der Amplifikationsreaktion mit einer Markierung versehen werden, kann aber auch erst nach Beendigung der Amplifikationsreaktion, also vor Analyse mittels des Nucleotid-Mikroarrays, markiert werden. Eine Markierung des Amplifikationsproduktes während der Amplifikationsreaktion kann beispielsweise unter Verwendung von markierten Primern und/oder markierten Nucleotiden erfolgen. Erfindungsgemäß kann das Amplifikationsprodukt zum Beispiel unter Verwendung von Fluoreszenz-Farbstoffen wie Cy3 oder Cy5, Biotin, Haptenen, Antigenen, chemischen Gruppen, beispielsweise durch Verwendung aminoallyl-markierter Nucleotide oder durch Anbringen eines Markers mittels einer Succinimid-Reaktion mit dem aminoallyl-markierten Nucleotid, radioaktiven Stoffen, enzymatischen Markern etc. markiert werden. Die Detektion des markierten Amplifikationsproduktes nach Hybridisierung des Amplifikationsproduktes mit einem komplementären Oligonucleotid oder Nucleinsäuremolekül an der Trägeroberfläche des Nucleotid-Arrays kann beispielsweise unter Verwendung von Fluoreszenz-Verfahren, Chemolumineszenz-Verfahren, radiometrischen Verfahren, Densitometrie-Verfahren, Photometrie-Verfahren, Fällungsreaktionen, enzymatischen Reaktionen einschließlich enzymatischen Verstärkungsreaktionen, SPR, Ellipsometrie-Verfahren, Messung des Brechungsindex, Messung der Reflektivität und ähnlichen Verfahren erfolgen.

In einer bevorzugten Ausführungsform können die OrientierungsKontrolle, die Hybridisierungs-Kontrolle und die Print-Kontrolle mittels Cy3 im Cy3-Kanal detektiert werden, während die Amplifikations-Kontrolle, die Proben-Kontrolle und die nachzuweisenden HPV-Genotypen mittels Cy5 im Cy5-Kanal detektiert werden.

Die vorliegende Erfindung betrifft ebenfalls einen Kit zum Nachweis und/oder zur Bestimmung genitaler HPV-Genotypen, umfassend mindestens einen ersten Behälter mit mindestens einem Primer zur Amplifikation von Nucleinsäurebereichen des HPV-Gens E1, und mindestens einen zweiten Behälter mit mindestens einer Sonde zum Nachweis und/oder Bestimmung genitaler HPV-Genotypen, insbesondere zum Nachweis eines amplifizierten Bereiches des HPV-Gens E1. Der zur Amplifikation verwendete Primer ist erfindungsgemäß ausgewählt aus Oligonucleotiden mit einer der in SEQ ID Nr. 1 bis 7 dargestellten Nucleotidsequenzen. Die Sonde zum Nachweis und/oder Bestimmung genitaler HPV-Genotypen ist ausgewählt aus Oligonucleotiden mit einer der in SEQ ID Nr. 8 bis 135 dargestellten Nucleotidsequenzen, vorzugsweise mit einer der in SEQ ID Nr. 19, 32, 41, 44, 48, 82 und 117 bis 135 dargestellten Nucleotidsequenzen, Oligonucleotiden mit einer Nucleotidsequenz, die gegenüber einer der in SEQ ID Nr. 8 bis 135 dargestellten Nucleotidsequenzen, vorzugsweise einer der in SEQ ID Nr. 19, 32, 41, 44, 48, 82 und 117 bis 135 dargestellten Nucleotidsequenzen, mutiert ist, Oligonucleotiden mit einer Nucleotidsequenz, die zu einer der in SEQ ID Nr. 8 bis 135 dargestellten Nucleotidsequenzen, vorzugsweise einer der in SEQ ID Nr. 19, 32, 41, 44, 48, 82 und 117 bis 135 dargestellten Nucleotidsequenzen, oder einer mutierten Nucleotidsequenz davon komplementär ist, oder Nucleinsäuremolekülen, die eine der in SEQ ID Nr. 8 bis 135 dargestellten Nucleotidsequenzen, vorzugsweise eine der in SEQ ID Nr. 19, 32, 41, 44, 48, 82 und 117 bis 135 dargestellten Nucleotidsequenzen, eine mutierte Nucleotidsequenz davon oder eine komplementäre Nucleotidsequenz davon umfassen.

In bevorzugter Ausführungsform der Erfindung umfasst der Kit mindestens 29 zweite Behälter mit mindestens 29 verschiedenen Sonden zum Nachweis und/oder zur Bestimmung der HPV6-, insbesondere HPV6b-, HPV11-, HPV16-, HPV18-, HPV26-, HPV31-, HPV33-, HPV34-, HPV35-, insbesondere HPV35h-, HPV39-, HPV40-; HPV42-, HPV43-, HPV44-, HPV45-, HPV51-, HPV52, HPV53-; HPV54-, HPV56-, HPV58-, HPV59-, HPV61-, HPV66-, HPV67-, HPV68-, HPV70-, HPV73- und HPV82-Genotypen, wobei jeder Behälter mindestens eine Sonde enthält und wobei alle in einem Behälter enthaltenen Sonden nur einen spezifischen HPV-Genotyp nachweisen können. Besonders bevorzugt umfasst der Kit mindestens 24 zweite Behälter mit mindestens 24 verschiedenen Sonden zum Nachweis und/oder zur Bestimmung der HPV6-, insbesondere HPV6b-, HPV11-, HPV16-, HPV18-, HPV31-, HPV33-, HPV35-, insbesondere HPV35h-, HPV39-, HPV40-, HPV42-, HPV43-, HPV44-, HPV45-, HPV51-, HPV52, HPV53-, HPV56-, HPV58-, HPV59-, HPV66-, HPV68-, HPV70-, HPV73- und HPV82-Genotypen.

Wenn beispielsweise die in einem Behälter enthaltenen Sonden zum Nachweis des HPV6-Genotyps, insbesondere HPV6b-Genotyps, verwendet werden sollen, kann der Behälter ein Oligonucleotid mit der in SEQ ID Nr. 8, 9 bzw. 117 dargestellten Nucleotidsequenz, vorzugsweise der in SEQ ID Nr. 117 dargestellten Nucleotidsequenz, ein Oligonucleotid mit einer Nucleotidsequenz, die gegenüber der Nucleotidsequenz von SEQ ID Nr. 8, 9 bzw. 117 mutiert ist, ein Oligonucleotid mit einer Nucleotidsequenz, die gegenüber SEQ ID Nr. 8, 9 bzw. 117 oder einer mutierten Sequenz davon komplementär ist, ein Nucleinsäuremolekül, das die Sequenz von SEQ ID Nr. 8, 9 bzw. 117, vorzugsweise die Sequenz von SEQ ID Nr. 117, eine mutierte Sequenz davon oder eine komplementäre Sequenz davon aufweist, oder ein Gemisch dieser Oligonucleotide und/oder Nucleinsäuremoleküle enthalten. Vorzugsweise enthält jeder Behälter jedoch kein Gemisch, sondern nur eine Oligonucleotid- oder Nucleinsäuremolekül-Spezies.

Die vorliegende Erfindung betrifft ebenfalls einen Kit zum Nachweis und/oder zur Bestimmung genitaler HPV-Genotypen, umfassend mindestens einen ersten Behälter mit mindestens einem Primer zur Amplifikation von Nucleinsäurebereichen des HPV-Gens E1, und einen Nucleotid-Array zum Nachweis und/oder Bestimmung genitaler HPV-Genotypen, insbesondere zum Nachweis eines amplifizierten Bereiches des HPV-Gens E1. Der zur Amplifikation verwendete Primer ist erfindungsgemäß ausgewählt aus Oligonucleotiden mit einer der in SEQ ID Nr. 1 bis 7 dargestellten Nucleotidsequenzen, den erfindungsgemäßen Primer-Paaren. Der Nucleotid-Array des Kits umfasst vorzugsweise Oligonucleotide mit einer der in SEQ ID Nr. 8 bis 135 dargestellten Nucleotidsequenzen, besonders bevorzugt einer der in SEQ ID Nr. 19, 32, 41, 44, 48, 82 und 117 bis 135 dargestellten Nucleotidsequenzen, Oligonucleotiden mit einer Nucleotidsequenz, die gegenüber einer der in SEQ ID Nr. 8 bis 135 dargestellten Nucleotidsequenzen, insbesondere einer der in SEQ ID Nr. 19, 32, 41, 44, 48, 82 und 117 bis 135 dargestellten Nucleotidsequenzen, mutiert ist, Oligonucleotiden mit einer Nucleotidsequenz, die zu einer der in SEQ ID Nr. 8 bis 135 dargestellten Nucleotidsequenzen, vorzugsweise einer der in SEQ ID Nr. 19, 32, 41, 44, 48, 82 und 117 bis 135 dargestellten Nucleotidsequenzen, oder einer mutierten Nucleotidsequenz davon komplementär ist, und/oder Nucleinsäuremoleküle, die eine der in SEQ ID Nr. 8 bis 135 dargestellten Nucleotidsequenzen, vorzugsweise eine der in SEQ ID Nr. 19, 32, 41, 44, 48, 82 und 117 bis 135 dargestellten Nucleotidsequenzen, eine mutierte Nucleotidsequenz davon oder eine komplementäre Nucleotidsequenz davon umfassen.

In bevorzugter Ausführungsform der Erfindung umfasst der erfindungsgemäße Kit mindestens zwei erste Behälter, wobei ein Behälter davon äquimolare Mengen der Oligonucleotide mit den in SEQ ID Nr. 2 bis 6 dargestellten Nucleotidsequenzen enthält und der andere Behälter das Oligonucleotid mit der in SEQ ID Nr. 7 dargestellten Nucleotidsequenz enthält. In einer alternativen Ausführungsform umfasst der erfindungsgemäße Kit sechs erste Behälter, wobei fünf Behälter davon jeweils eines der Oligonucleotide mit den in SEQ ID Nr. 2 bis 6 dargestellten Nucleotidsequenzen enthalten und der sechste Behälter das Oligonucleotid mit der in SEQ ID Nr. 7 dargestellten Nucleotidsequenz enthält.

Der erfindungsgemäße Kit kann zusätzlich einen Behälter mit einer Kontroll-Nucleinsäure umfassen, die unter Verwendung eines Oligonucleotids mit einer der in SEQ ID Nr. 1 bis 6 dargestellten Nucleotidsequenzen als Forward-Primer und eines Oligonucleotids mit der in SEQ ID Nr. 7 dargestellten Nucleotidsequenz als Reverse-Primer amplifiziert werden kann.

Die vorliegende Offenbarung betrifft selbstverständlich auch die Verwendung eines Oligonucleotids mit einer der in SEQ ID Nr. 8 bis 135 dargestellten Nucleotidsequenzen, vorzugsweise einer der in SEQ ID Nr. 19, 32, 41, 44, 48, 82 und 117 bis 135 dargestellten Nucleotidsequenzen, eines Oligonucleotids, dessen Nucleotidsequenz gegenüber einer der in SEQ ID Nr. 8 bis 135 dargestellten Nucleotidsequenzen, insbesondere einer der in SEQ ID Nr. 19, 32, 41, 44, 48, 82 und 117 bis 135 dargestellten Nucleotidsequenzen mutiert ist, eines Oligonucleotids, dessen Nucleotidsequenz zu einer der in SEQ ID Nr. 8 bis 135 dargestellten Nucleotidsequenzen, insbesondere einer der in SEQ ID Nr. 19, 32, 41, 44, 48, 82 und 117 bis 135 dargestellten Nucleotidsequenzen, oder einer mutierten Nucleotidsequenz davon komplementär ist, oder eines Nucleinsäuremoleküls, das eine der in SEQ ID Nr. 8 bis 135 dargestellten Nucleotidsequenzen, insbesondere eine der in SEQ ID Nr. 19, 32, 41, 44, 48, 82 und 117 bis 135 dargestellten Nucleotidsequenzen, eine mutierte Sequenz davon oder eine komplementäre Sequenz davon umfasst, zum Nachweis und/oder zur Bestimmung eines genitalen HPV-Genotyps, das heißt zur Typisierung eines Papillomavirus, dessen Vorhandensein in einer biologischen Probe vorzugsweise zuvor bereits nachgewiesen wurde.

Die vorliegende Erfindung betrifft ebenfalls die Verwendung eines Oligonucleotids mit einer der in SEQ ID Nr. 1 bis 7 dargestellten Nucleotidsequenzen oder eines erfindungsgemäßen Primer-Paares zur Amplifikation eines Nucleinsäure-Bereiches eines genitalen humanen Papilloma-Virus, wobei in bevorzugter Ausführungsform der zu amplifizierende Nucleinsäure-Bereich ein Bereich des HPV-Gens E1 ist.

Die vorliegende Offenbarung betrifft auch die Verwendung eines Oligonucleotids mit einer der in SEQ ID Nr. 1 bis 135 dargestellten Nucleotidsequenzen, eines Oligonucleotids, dessen Nucleotidsequenz gegenüber einer der in SEQ ID Nr. 1 bis 135 dargestellten Nucleotidsequenzen mutiert ist, eines Oligonucleotides, das eine Nucleotidsequenz aufweist, die zu einer der in SEQ ID Nr. 8 bis 135 dargestellten Nucleotidsequenzen oder einer mutierten Sequenz davon komplementär ist, eines Nucleinsäuremoleküles, das eine der in SEQ ID Nr. 8 bis 135 dargestellten Nucleotidsequenzen, eine mutierte Sequenz davon oder eine komplementäre Sequenz davon umfasst, oder eines erfindungsgemäßen Primer-Paares zur Diagnose und/oder Früherkennung von Krankheiten, die von genitalen humanen Papillomaviren verursacht werden.

Die vorliegende Offenbarung betrifft ebenfalls die Verwendung eines Oligonucleotids mit einer der in SEQ ID Nr. 1 bis 135 dargestellten Nucleotidsequenzen, insbesondere einer der in SEQ ID Nr. 1 bis 7, 19, 32, 41, 44, 48, 82 und 117 bis 135 dargestellten Nucleotidsequenzen, eines Oligonucleotids, dessen Nucleotidsequenz gegenüber einer der in SEQ ID Nr. 1 bis 135 dargestellten Nucleotidsequenzen, insbesondere einer der in SEQ ID Nr. 1 bis 7, 19, 32, 41, 44, 48, 82 und 117 bis 135 dargestellten Nucleotidsequenzen, mutiert ist, eines Oligonucleotides, das eine Nucleotidsequenz aufweist, die zu einer der in SEQ ID Nr. 8 bis 135 dargestellten Nucleotidsequenzen, insbesondere einer der in SEQ ID Nr. 1 bis 7, 19, 32, 41, 44, 48, 82 und 117 bis 135 dargestellten Nucleotidsequenzen, oder einer mutierten Sequenz davon komplementär ist, eines Nucleinsäuremoleküles, das eine der in SEQ ID Nr. 8 bis 135 dargestellten Nucleotidsequenzen, insbesondere eine der in SEQ ID Nr. 19, 32, 41, 44, 48, 82 und 117 bis 135 dargestellten Nucleotidsequenzen, eine mutierte Sequenz davon oder eine komplementäre Sequenz davon umfasst, oder eines erfindungsgemäßen Primer-Paares zur Herstellung eines Mittels zur Diagnose von Krankheiten, die von genitalen humanen Papillomaviren verursacht werden.

Bei dem herzustellenden Mittel kann es sich beispielsweise um einen erfindungsgemäßen Kit oder einen erfindungsgemäßen Nucleotid-Array handeln.

Die vorliegende Erfindung wird durch das folgende Sequenzprotokoll, die folgenden Figuren und die folgenden Beispiele näher erläutert.

Das Sequenzprotokoll ist Teil dieser Beschreibung und enthält die Sequenzen SEQ ID Nr. 1 bis 135.

SEQ ID Nr. 1 bis SEQ ID Nr. 6 zeigen die Sequenzen von Oligonucleotiden, die als Forward-Primer zur Amplifikation von Bereichen des HPV-Gens E1 geeignet sind. Die als Forward-Primer verwendeten Oligonucleotide mit den in SEQ ID Nr. 2 bis 6 dargestellten Nucleotidsequenzen werden im Folgenden auch als Loma 1, Loma 2, Loma 3, Loma 4 beziehungsweise Loma 5 bezeichnet.

SEQ ID Nr. 7 zeigt die Sequenz eines Oligonucleotids, das als Reverse-Primer zur Amplifikation von Bereichen des HPV-Gens E1 geeignet ist. Das als Reverse-Primer verwendete Oligonucleotid mit der in SEQ ID Nr. 7 dargestellten Nucleotidsequenz wird im Folgenden auch als Loma-rev bezeichnet.

SEQ ID Nr. 8, 9 und 117 zeigen die Sequenzen von Oligonucleotiden, die zum Nachweis und zur Bestimmung des HPV6-Genotyps, insbesondere HPV6b-Genotyps geeignet sind.

SEQ ID Nr. 10 bis SEQ ID Nr. 17 und SEQ ID Nr. 118 zeigen die Sequenzen von Oligonucleotiden, die zum Nachweis und zur Bestimmung des HPV11-Genotyps geeignet sind.

SEQ ID Nr. 18 bis SEQ ID Nr. 20 zeigen die Sequenzen von Oligonucleotiden, die zum Nachweis und zur Bestimmung des HPV16-Genotyps geeignet sind.

SEQ ID Nr. 21 bis SEQ ID Nr. 24 und SEQ ID Nr. 119 zeigen die Sequenzen von Oligonucleotiden, die zum Nachweis und zur Beistimmung des HPV18-Genotyps geeignet sind.

SEQ ID Nr. 25 bis SEQ ID Nr. 28 zeigen die Sequenzen von Oligonucleotiden, die zum Nachweis und zur Bestimmung des HPV26-Genotyps geeignet sind.

SEQ ID Nr. 29 bis SEQ ID Nr. 31 und SEQ ID Nr. 120 zeigen die Sequenzen von Oligonucleotiden, die zum Nachweis und zur Bestimmung des HPV31-Genotyps geeignet sind.

SEQ ID Nr. 32 bis SEQ ID Nr. 34 zeigen die Sequenzen von Oligonucleotiden, die zum Nachweis und zur Bestimmung des HPV33-Genotyps geeignet sind.

SEQ ID Nr. 35 bis SEQ ID Nr. 39 zeigen die Sequenzen von Oligonucleotiden, die zum Nachweis und zur Bestimmung des HPV34-Genotyps geeignet sind.

SEQ ID Nr. 40 und SEQ ID Nr. 41 zeigen die Sequenzen von Oligonucleotiden, die zum Nachweis und zur Bestimmung des HPV35-Genotyps, insbesondere HPV35h-Genotyps geeignet sind.

SEQ ID Nr. 42 bis SEQ ID Nr. 46 zeigen die Sequenzen von Oligonucleotiden, die zum Nachweis und zur Bestimmung des HPV39-Genotyps geeignet sind.

SEQ ID Nr. 47 bis SEQ ID Nr. 50 zeigen die Sequenzen von Oligonucleotiden, die zum Nachweis und zur Bestimmung des HPV40-Genotyps geeignet sind.

SEQ ID Nr. 51, SEQ ID Nr. 52 und SEQ ID Nr. 121 zeigen die Sequenzen von Oligonucleotiden, die zum Nachweis und zur Bestimmung des HPV42-Genotyps geeignet sind.

SEQ ID Nr. 122 zeigt die Sequenz eines Oligonucleotids, das zum Nachweis und zur Bestimmung des HPV43-Genotyps geeignet ist.

SEQ ID Nr. 53 bis SEQ ID Nr. 55 und SEQ ID Nr. 123 zeigen die Sequenzen von Oligonucleotiden, die zum Nachweis und zur Bestimmung des HPV44-Genotyps geeignet sind.

SEQ ID Nr. 56 bis SEQ ID Nr. 61 und SEQ ID Nr. 124 zeigen die Sequenzen von Oligonucleotiden, die zum Nachweis und zur Bestimmung des HPV45-Genotyps geeignet sind.

SEQ ID Nr. 62 bis SEQ ID Nr. 64 und SEQ ID Nr. 125 zeigen die Sequenzen von Oligonucleotiden, die zum Nachweis und zur Bestimmung des HPV51-Genotyps geeignet sind.

SEQ ID Nr. 65 bis SEQ ID Nr. 67 und SEQ ID Nr. 126 zeigen die Sequenzen von Oligonucleotiden, die zum Nachweis und zur Bestimmung des HPV52-Genotyps geeignet sind.

SEQ ID Nr. 68 bis SEQ ID Nr. 73 und SEQ ID Nr. 127 zeigen die Sequenzen von Oligonucleotiden, die zum Nachweis und zur Bestimmung des HPV53-Genotyps geeignet sind.

SEQ ID Nr. 74 bis SEQ ID Nr. 77 zeigen die Sequenzen von Oligonucleotiden, die zum Nachweis und zur Bestimmung des HPV54-Genotyps geeignet sind.

SEQ ID Nr. 78 bis SEQ ID Nr. 81 und SEQ ID Nr. 128 zeigen die Sequenzen von Oligonucleotiden, die zum Nachweis und zur Bestimmung des HPV56-Genotyps geeignet sind.

SEQ ID Nr. 82 bis SEQ ID Nr. 86 zeigen die Sequenzen von Oligonucleotiden, die zum Nachweis und zur Bestimmung des HPV58-Genotyps geeignet sind.

SEQ ID Nr. 87 und SEQ ID Nr. 129 zeigen die Sequenzen von Oligonucleotiden, die zum Nachweis und zur Bestimmung des HPV59-Genotyps geeignet sind.

SEQ ID Nr. 88 bis SEQ ID Nr. 94 zeigen die Sequenzen von Oligonucleotiden, die zum Nachweis und zur Bestimmung des HPV61-Genotyps geeignet sind.

SEQ ID Nr. 95 bis SEQ ID Nr. 97 und SEQ ID Nr. 130 zeigen die Sequenzen von Oligonucleotiden, die zum Nachweis und zur Bestimmung des HPV66-Genotyps geeignet sind.

SEQ ID Nr. 98 bis SEQ ID Nr. 102 zeigen die Sequenzen von Oligonucleotiden, die zum Nachweis und zur Bestimmung des HPV67-Genotyps geeignet sind.

SEQ ID Nr. 103 bis SEQ ID Nr. 105, SEQ ID Nr. 131 und SEQ ID Nr. 132 zeigen die Sequenzen von Oligonucleotiden, die zum Nachweis und zur Bestimmung des HPV68-Genotyps geeignet sind.

SEQ ID Nr. 106 bis SEQ ID Nr. 108 und SEQ ID Nr. 133 zeigen die Sequenzen von Oligonucleotiden, die zum Nachweis und zur Bestimmung des HPV70-Genotyps geeignet sind.

SEQ ID Nr. 109 bis SEQ ID Nr. 112 und SEQ ID Nr. 134 zeigen die Sequenzen von Oligonucleotiden, die zum Nachweis und zur Bestimmung des HPV73-Genotyps geeignet sind.

SEQ ID Nr. 113 bis SEQ ID Nr. 116 und SEQ ID Nr. 135 zeigen die Sequenzen von Oligonucleotiden, die zum Nachweis und zur Bestimmung des HPV82-Genotyps geeignet sind.

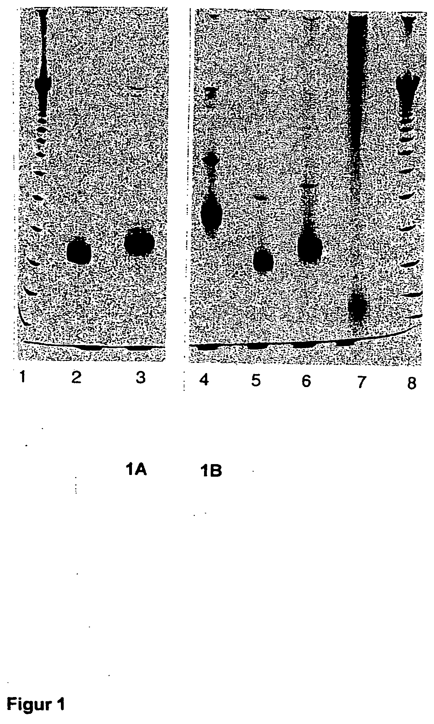

Figur 1 zeigt die Ergebnisse einer Acrylamid-Gelelektrophorese (nicht-denaturierend) von Amplifikationsprodukten, die unter Verwendung der erfindungsgemäßen Primer und der DNA verschiedener Papilloma-Viren als Matrize erhalten wurden. Als Forward-Primer wurde ein äquimolares Gemisch der Primer Loma 1, Loma 2, Loma 3, Loma 4 und Loma 5 (die den Oligonucleotiden mit den in SEQ ID Nr. 2 bis 6 dargestellten Nucleotidsequenzen entsprechen) eingesetzt. Als Reverse-Primer wurde der Primer Loma-rev (entspricht dem Oligonucleotid mit der in SEQ ID Nr. 7 dargestellten Nucleotidsequenz) eingesetzt. Als Matrize wurden Plasmide verwendet, die das gesamte HPV6-, HPV16-, HPV58-, HPV59- beziehungsweise HPV82-Genom enthielten. Als Negativ-Kontrolle wurde humane DNA verwendet.

Figur 1A: Nucleinsäure-Größenstandard (Laufspur 1), Amplifikationsprodukt unter Verwendung von HPV6-DNA (Laufspur 2), Amplifikationsprodukt unter Verwendung von HPV16-DNA (Laufspur 3),

Figur 1B: Amplifikationsprodukt unter Verwendung von HPV58-DNA (Laufspur 4), Amplifikationsprodukt unter Verwendung von HPV59-DNA (Laufspur 5), Amplifikationsprodukt unter Verwendung von HPV82-DNA (Laufspur 6), Amplifikationsreaktion unter Verwendung humaner-DNA (Laufspur 7; Negativ-Kontrolle), Nucleinsäure-Größenstandard (Laufspur 8).

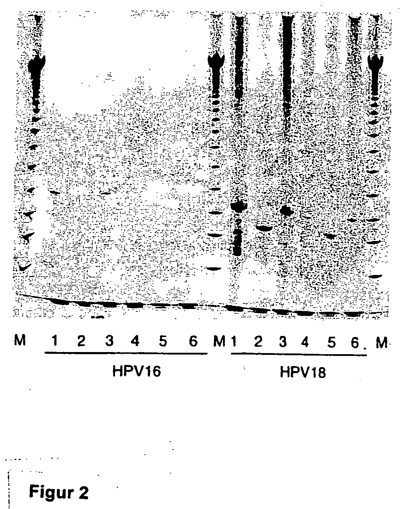

Figur 2 zeigt die Ergebnisse einer Acrylamid-Gelelektrophorese (nicht-denaturierend) von Amplifikationsprodukten, die unter Verwendung von HPV16- oder HPV18-DNA als Matrize erhalten wurden. Als Primer wurden die erfindungsgemäße Primer-Kombination Loma1-5/Loma-rev (Kombination 1), die Primer-Kombination Loma1-5/P2 (SEQ ID Nr. 2 aus DE 100 09 143 A1) (Kombination 2), die Primer-Kombination Loma1-5/P3 (SEQ ID Nr. 3 aus DE 100 09 143 A1) (Kombination 3), die Primer-Kombination P1 (SEQ ID Nr. 1 aus DE 100 09 143 A1)/Loma-rev (Kombination 4), die Primer-Kombination P1/P2 (Kombination 5) und die Primer-Kombination P1/P3 (Kombination 6) eingesetzt. M = DNA-Größenstandard (123 bp-Leiter), 1 = Primer-Kombination 1, 2 = Primer-Kombination 2, 3 = Primer-Kombination 3, 4 = Primer-Kombination 4, 5 = Primer-Kombination 5, 6 = Primer-Kombination 6.

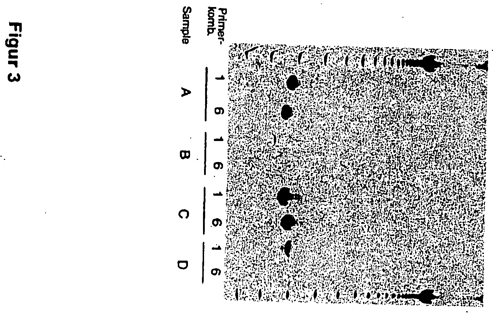

Figur 3 zeigt die Ergebnisse einer Acrylamid-Gelelektrophorese (nicht-denaturierend) von Amplifikationsprodukten, die unter Verwendung von Patientenproben erhalten wurden. Als Primer wurden die Primer-Kombinationen 1 (Loma1-5/Loma-rev) und 6 (P1/P3) eingesetzt. A = Patientenprobe, die mittels in situ-Hybridisierung als HPV16-positiv eingestuft wurde, B = Patientenprobe, die mittels in situ-Hybridisierung als HPV73-positiv eingestuft wurde, C = Patientenprobe, die mittels in situ-Hybridisierung als negativ eingestuft wurde, D = Patientenprobe, die mittels in situ-Hybridisierung als HPV33-positiv eingestuft wurde. In den Laufspuren links außen und rechts außen ist jeweils ein DNA-Größenstandard (123 bp-Leiter) aufgetragen.

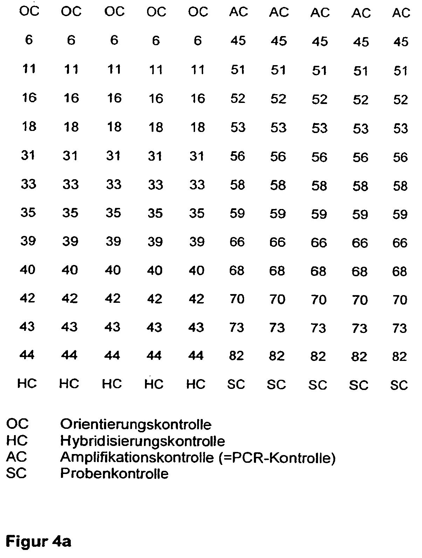

Figur 4a zeigt das Design eines erfindungsgemäßen Nucleotid-Arrays oder Nucleotid-Chips, wobei auf der Oberfläche des Microarray-Trägers eine Kontrolle zur Orientierung des Microarray-Trägers (OC), eine Hybridisierungskontrolle (HC), eine PCR-Kontrolle (AC), eine Proben-Kontrolle (Sample-Kontrolle, SC) sowie Sonden zur Typisierung von HPV-Genotypen angeordnet sind. Der Microarray weist darüber hinaus Spots zur Chip-Codierung auf. Die Hybridisierungskontrolle und PCR-Kontrolle können jeweils als Verdünnungsreihen der entsprechenden Oligonucleotide ausgeführt sein. Zahlen geben die Bezeichnungen des HPV-Typen an, für die die jeweilige Sonde spezifisch ist. Eine Printkontrolle ist zusätzlich auf allen Spots außer der Orientierungskontrolle und der Hybridisierungskontrolle angebracht.

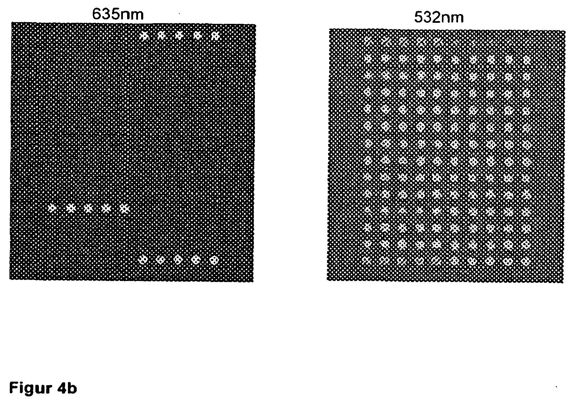

Figur 4b zeigt eine konkret durchgeführte Hybridisierung für HPV42. OC, HC und Printkontrollen werden durch Fluoreszenzlicht nach Anregung mit Licht der Wellenlänge 532nm detektiert. AC, SC und die HPC-spezifischen Signale durch Fluoreszenzlicht nach Anregung mit Licht der Wellenlänge 635nm.

### Beispiel 1

### Amplifikation von HPV-DNA unter Verwendung der erfindungsgemäßen Primer Loma/Loma-rev

Zur Amplifikation wurden Plasmide mit den Genomen von HPV6, HPV16, HPV58, HPV59 und HPV82 als Matrize (Template) verwendet. Diese Plasmide enthalten das gesamte Genom des entsprechenden HPV-Typs. Als Negativ-Kontrolle wurde humane DNA verwendet. Tabelle 1 zeigt die Zusammensetzung des Reaktionsansatzes und die Konzentration der eingesetzten Primer.

**Tabelle 1**

| Lösung | µl | Endkonzentration |
|---|---|---|
| H₂O | 12,6 | |
| 10 x PCR-Buffer (für AmpliTaq Gold; ohne MgCl₂ | 2 | 1x |
| 25 mM MgCl₂ | 2 | 2,5 mM |
| 25 mM dNTP (f.c.: 0,25 mM) | 0,2 | 0,25 mM |
| Loma-Mix mit 4 pMol/µl von jedem der Primer Loma1-Loma5 | 1 | 0,2 pMol/µl von jedem der 5 Loma Primer |
| 20 pMol/µl Loma-rev Cy5-markiert | 1 | 1 pMOl/µl |
| 5 u/µl Ampli Taq Gold | 0,2 | 0,05 u/µl |
| DNA-Template (HPV-Plasmid) | 1 | |
| Summe | 20 | |

Die PCR wurde unter folgenden Temperaturbedingungen durchgeführt. Zunächst wurde der Reaktionsansatz auf eine Temperatur auf 95°C gebracht, wobei die Temperatur mit einer Geschwindigkeit von 1°C/sec erhöht wurde. Die Temperatur des Reaktionsansatzes wurde 10 Minuten bei 95°C gehalten. Danach wurde der Reaktionsansatz folgenden Temperaturbedingungen unterworfen: pro Zyklus 30 Sekunden bei 95°C, 30 Sekunden bei 55°C und 1 Minute bei 72°C. Insgesamt wurden 40 Zyklen durchgeführt. Nach Durchführung der 40 Zyklen wurde die Temperatur des Reaktionsansatzes 5 Minuten bei 72°C gehalten und dann auf 4°C abgekühlt.

Jeweils 2 µl der erhaltenen PCR-Produkte wurde auf einem nicht-denaturierenden Acrylamid-Gel aufgetrennt und mittels Silber-Färbung sichtbar gemacht. Die Ergebnisse sind in den Figuren 1A und 1B dargestellt. Sequenzunterschiede zwischen den verschiedenen HPV-Typen führen zu einem unterschiedlichen Laufverhalten im nicht-denaturierenden Gel. Wie aus Figur 1 ersichtlich, wurde bei der Kontroll-DNA als Negativ-Kontrolle kein PCR-Produkt erhalten.

### Beispiel 2

### PCR-Amplifikation unter Verwendung der erfindungsgemäßen Primer im Vergleich zur PCR-Amplifikation unter Verwendung von im Stand der Technik bekannten Primern

In diesem Versuch sollte die Eignung der erfindungsgemäßen Forward- und Reverse-Primer bei der Amplifikation von Nucleinsärue-Bereichen genitaler HPV-Viren im Vergleich zu der PCR-Amplifikation unter Verwendung von aus dem Stand der Technik bekannten Primerpaaren bestimmt werden.

In der DE 100 09 143 A1 werden zwei PCR-Primersysteme beschrieben. Diese Primersysteme umfassen entweder den Primer mit SEQ ID Nr. 1 (im Folgenden auch als P1 bezeichnet) und mit SEQ ID Nr. 2 (im Folgenden auch als P2 bezeichnet) oder der Primer mit SEQ ID Nr. 1 und SEQ ID Nr. 3 (im Folgenden auch als P3 bezeichnet). Da nur das PCR-System mit den Primern P1 und P3 zur Typisierung von Papilloma-Viren geeignet sind, stellt dieses die maßgebende Vergleichsbasis für das erfindungsgemäße Primersystem Loma/Loma-rev dar. Aufgrund der Lage der Primer am HPV-Genom können sowohl der Primer P1 als auch das erfindungsgemäße äquimolare Primer-Gemisch, umfassend die Primer Loma 1, Loma 2, Loma 3, Loma 4 und Loma 5, mit jedem der Primer P2, P3 und Loma-rev kombiniert werden, um ein PCR-Produkt zu erhalten. In dem Versuch wurden alle sechs möglichen Primer-Kombinationen ausgetestet. Die verwendeten Primer-Kombinationen sind in Tabelle 2 dargestellt.

Als DNA-Templates wurden das HPV16-Plasmid mit dem Genom des HPV16-Typs sowie HPV18-DNA, die aus HeLa-Zellen extrahiert worden war, eingesetzt. Die Konzentrationen der jeweiligen Templates wurden in Vorversuchen so eingestellt, dass sie in der Nähe der Grenze der Amplifizierbarkeit mit dem Primersystem Loma/Loma-rev kommen, aber noch ein deutliches PCR-Amplifikationsprodukt ergeben. Die PCR wurde unter folgenden Temperaturbedingungen durchgeführt: Rampe: 1°C/sec, Halten der Temperatur bei 95°C über 10 min, 40 Zyklen mit jeweils 30 sec bei 95°C, 30 sec bei 55°C, 1 min bei 72°C, danach 5 min Halten der Temperatur bei 72°C und Abkühlen auf 4°C.

**Tabelle 2**

| Primer-kombination | Forward-Primer | Reverse-Primer |
|---|---|---|
| 1 | Loma 1-5 | Loma-rev |
| 2 | Loma 1-5 | SEQ ID Nr. 2 aus DE 100 09 143 (P2) |
| 3 | Loma 1-5 | SEQ ID Nr. 3 aus DE 100 09 143 (P3) |
| 4 | SEQ ID Nr. 1 aus DE 100 09 143 (P1) | Loma-rev |
| 5 | SEQ ID Nr. 1 aus DE 100 09 143 (P1) | SEQ ID Nr. 2 aus DE 100 09 143 (P2) |
| 6 | SEQ ID Nr. 1 aus DE 100 09 143 (P1) | SEQ ID Nr. 3 aus DE 100 09 143 (P3) |

Die mit den vorstehenden Primer-Kombinationen erhaltenen Ergebnissse sind in Figur 2 dargestellt. Die Primer-Kombination 1 ergibt sowohl bei der Amplifikation der HPV16-DNA als auch bei der Amplifikation der HPV18-DNA ein PCR-Produkt. Die Primer-Kombination 2 ergibt im Falle der HPV16-DNA kein nachweisbares PCR-Produkt. Die Primer-Kombination 3 ergibt bei beiden getesteten Nucleinsäure-Templates ein PCR-Produkt. Die Primer-Kombinationen 4 bis 6 ergeben bei der gewählten Verdünnung der HPV16-DNA kein PCR-Produkt und bei der HPV18-DNA eine deutlich geringere Amplifikationsprodukt-Menge als die Primer-Kombinationen 1 bis 3.

### Beispiel 3

### Amplifikation von HPV-Templates aus Patientinnen-Proben

Unter Verwendung von DNA-Extrakten aus vier Proben von Patientinnen (Paraffinschnitte von Cervicalproben) wurden PCR-Reaktionen mit den Primer-Kombinationen 1 und 6 (vergleiche Tabelle 2) durchgeführt. Diese Proben waren zuvor mittels in situ-Hybridisierung auf HPV-Infektionen untersucht worden. Die Ergebnisse sind in Tabelle 3 und Figur 3 dargestellt.

**Tabelle 3**

| Patientenprobe | Ergebnis In-situ Hybridisierung | Ergebnis PCR Loma1-5/ Loma rev | Ergebnis PCR SEQ ID Nr. 1 (P1)/ SEQ ID Nr. 3 (P3) |
|---|---|---|---|
| A | HPV16 | + | + |
| B | HPV73 | + | - |
| C | Negativ | + | + |
| D | HPV33 | + | - |

Aus den Ergebnissen ist erkennbar, dass beide PCR-Reaktionen im Fall der Probe C sensitiver als die Hybridisierung sind, da diese Probe bei beiden Primerpaaren positiv war. Die Proben B und D sind nur mit den erfindungsgemäßen Primern Loma/Loma-rev positiv, nicht jedoch mit der aus dem Stand der Technik bekannten Primer-Kombination P1/P3.

### Beispiel 4

### Hybridisierung eines Amplifikationsprodukts der Loma-Primer auf einen erfindungsgemäßen Nucleotidarray

Zur Amplifikation wurde als Template ein Plasmid mit einem Teil des Genoms von HPV42 eingesetzt, gemischt mit humaner DNA und mit einem Plasmid, das ein synthetisches DNA-Konstrukt, das einen kurzen Ausschnitt aus dem Genom des Lambda-Phagen flankiert von Bindungsstellen für die Primer nach SEQ ID Nr. 4 und SEQ ID Nr. 7, enthält. Die Sonde der Amplifikationskontrolle am Nucleotidarray ist spezifisch für diesen Ausschnitt des Lambda-Phagen-Genoms.

Im PCR-Ansatz wurden ausser den Loma Primern auch Primer zur Amplifikation eines Ausschnitts aus dem humanen ADAT1-Gen verwendet.

Einer der Primer zur Amplifikation des humanen ADAT1-Gens und der Primer nach SEQ ID Nr. 6 sind mit dem Fluoreszenzfarbstoff Cy5 markiert.

Nach erfolgter DNA-Amplifikation wurden 5µl des PCR-Produkts mit 30µl Hybridisierungspuffer (0.5% Laurylsarcosin, 0.225M NaCl, 0.0225M Natrium-Citrat, 20nm eines Cy3-markierten Oligonukleotids, das komplementär zur Hybridisierungs- und Printkontrolle am Nucleotidarray ist) gemischt. Die Mischung wurde 3min bei 95°C denaturiert, auf Eiswasser abgekühlt und für 10min bei 50°C auf den Nucleotidarray hybridisiert. Der Nucleotidarray wurde 3x für jeweils 20 Sekunden bei 50°C in Waschpuffer (0.2% Natriumdodecylsulfat, 0.3M NaCl, 0.03M Natrium Citrat, pH7) gewaschen. In einem handelsüblichen Microarrayscanner wurde der Nucleotidarray bei Anregung mit Licht einer Wellenlänge von 532nm und 635nm gescannt. Das Ergebnis ist in Figur 4b gezeigt. Figur 4a zeigt die Position der Sonden auf dem verwendeten Nucleotidarray.

## Patentansprüche

1. Oligonucleotid, das als Forward-Primer zur Amplifikation eines Nucleinsäure-Bereiches eines genitalen humanen Papillomavirus (HPV) eingesetzt werden kann und das die Sequenz 5'-CAR GCI AAA WWW KTD AAR GAY TGT G-3' oder 5'-CAR GCN AAA WWW KTD AAR GAY TGT G-3' (SEQ ID Nr. 1) aufweist, wobei R = A oder G ist, W = T oder A ist, K = T oder G ist, I = Inosin ist, N = A, T, G oder C ist, D = A, T oder G ist und Y = C oder T ist.

2. Oligonucleotid nach Anspruch 1, wobei das Oligonucleotid ausgewählt ist aus der Gruppe bestehend aus:
a) einem Oligonucleotid mit der Nucleotidsequenz 5'-CAR GCI AAA TAT KTR AAA GAT TGT G-3' oder 5'-CAR GCN AAA TAT KTR AAA GAT TGT G-3' (SEQ ID Nr. 2),
b) einem Oligonucleotid mit der Nucleotidsequenz 5'-CAR GCA AAA TAT GTW AAG GAT TGT G-3 '(SEQ ID Nr. 3),
c) einem Oligonucleotid mit der Nucleotidsequenz 5'-CAR GCW AAA ATT GTA AAR GAT TGT G-3' (SEQ ID Nr. 4),
d) einem Oligonucleotid mit der Nucleotidsequenz 5'-CAA GCA AAA ATA GTA AAR GAC TGT G-3' (SEQ ID Nr. 5), und
e) einem Oligonucleotid mit der Nucleotidsequenz 5'-CAR GCA AAA TAT GTA AAA GAC TGT G-3' (SEQ ID Nr. 6),
wobei R = A oder G ist, W = T oder A ist, K = T oder G ist, I = Inosin ist und N = A, T, G oder C ist.

3. Gemisch aus Oligonucleotiden, das als Reverse-Primer zur Amplifikation eines Nucleinsäure-Bereiches eines genitalen humanen Papillomavirus eingesetzt werden kann, mit der Nucleotidsequenz 5'-ARY GGY TSY ARC CAA AAR TGR CT-3' (SEQ ID Nr. 7), wobei R = A oder G ist, Y = C oder T ist und S = C oder G ist.

4. Primer-Paar zur Amplifikation eines Nucleinsäure-Bereiches eines genitalen humanen Papillomavirus (HPV), umfassend einen Forward-Primer und einen Reverse-Primer, wobei der Forward-Primer ausgewählt ist aus der Gruppe bestehend aus:
a) einem Oligonucleotid nach Anspruch 1 oder 2 mit einer der in SEQ ID Nr. 1 bis 6 dargestellten Nucleotidsequenzen, und
b) einem Gemisch der Oligonucleotide nach a),
und der Reverse-Primer das Oligonucleotid mit der in SEQ ID Nr. 7 dargestellten Nucleotidsequenz ist.

5. Primer-Paar nach Anspruch 4, wobei der Forward-Primer ein äquimolares Gemisch der Oligonucleotide mit den in SEQ ID Nr. 2 bis 6 dargestellten Nucleotidsequenzen und der Reverse-Primer das Oligonucleotid mit der in SEQ ID Nr. 7 dargestellten Nucleotidsequenz ist.

6. Verfahren zur Amplifikation eines Bereiches einer in einer biologischen Probe vorhandenen Nucleinsäure eines genitalen humanen Papillomavirus, umfassend die Durchführung eines Nucleinsäure-Amplifikationsverfahrens unter Verwendung eines einen Forward-Primer und einen Reverse-Primer umfassenden Primer-Paares, wobei der Forward-Primer ausgewählt ist aus der Gruppe bestehend aus:
a) einem Oligonucleotid nach Anspruch 1 oder 2 mit einer der in SEQ ID Nr. 1 bis 6 dargestellten Nucleotidsequenzen, und
b) einem Gemisch der Oligonucleotide nach a),
und der Reverse-Primer das Oligonucleotid mit der in SEQ ID Nr. 7 dargestellten Nucleotidsequenz ist.

7. Verfahren nach Anspruch 6, wobei das Nucleinsäureamplifikations-Verfahren ein PCR (polymerase chain reaction)-Verfahren ist und die Nucleinsäureamplifikation unter folgenden Temperatur-Bedingungen durchgeführt wird:
a) Erhitzen auf 95°C, wobei die Temperatur pro sec um 1°C erhöht wird,
b) Halten der Temperatur bei 95°C für 10 min,
c) Durchführung von 40 Zyklen, jeweils umfassend 30 sec bei 95°C, 30 sec bei 55°C und 1 min bei 72°C,
d) Halten der Temperatur bei 72°C für 5 min und
e) Abkühlen auf 4°C.

8. Verfahren nach einem der Ansprüche 6 oder 7, wobei das Amplifikationsprodukt während oder nach der Amplifikationsreaktion mit einer Markierung versehen wird.

9. Verfahren zum Nachweis und/oder zur Bestimmung eines genitalen HPV-Genotyps, umfassend die Untersuchung des in einer biologischen Probe vorhandenen HPV-Gens E1 oder eines Teils davon eines genitalen humanen Papillomavirus durch Hybridisierung mit mindestens einer Sonde, wobei die Sonde ausgewählt ist aus der Gruppe bestehend aus:
a) HPV-Genotyp-spezifischen Oligonucleotiden mit den in SEQ ID Nr. 8 bis 135 dargestellten Nucleotidsequenzen,
b) Oligonucleotiden, die eine gegenüber einem der Oligonucleotide nach a) mutierte Nucleotidsequenz aufweisen, nämlich eine Deletion oder Addition von 1 bis 10 Nucleotiden oder eine Substitution von 1 bis 3 Nucleotiden in einer der in a) dargestellten Nucleotidsequenzen,
c) Oligonucleotiden, die eine Nucleotidsequenz aufweisen, die über die gesamte Länge zu der Nucleotidsequenz eines Oligonucleotids nach a) oder b) komplementär ist,
d) Nucleinsäuremolekülen, umfassend mindestens einen Bereich, der eine der in a) bis c) dargestellten Nucleotidsequenzen und einen oder mehrere zusätzliche Bereiche mit einer Gesamtlänge von mindestens einem Nucleotid aufweist, und
e) Gemischen der Oligonucleotide nach a) bis c) und/oder der Nucleinsäuremoleküle nach d),
und den Nachweis der Hybridisierung und wobei vor Hybridisierung mit der Sonde die in der biologischen Probe vorhandene HPV-Nucleinsäure amplifiziert wird und die Amplifikation der Nucleinsäure mittels eines Verfahrens nach einem der Ansprüche 6 bis 8 erfolgt.

10. Kit zum Nachweis und/oder zur Bestimmung genitaler HPV-Genotypen, umfassend mindestens einen ersten Behälter mit mindestens einem Primer zur Amplifikation von Bereichen des HPV-Gens E1, ausgewählt aus Oligonucleotiden nach einem der Ansprüche 1 oder 2, einem Gemisch aus Oligonucleotiden nach Anspruch 3 und/oder Primer-Paaren nach Anspruch 4 oder 5, und mindestens einen zweiten Behälter mit mindestens einer Sonde zum Nachweis eines amplifizierten Bereiches des HPV-Gens E1, ausgewählt aus Oligonucleotiden mit einer der in SEQ ID Nr. 8 bis 135 dargestellten Nucleotidsequenzen, mit einer mutierten Sequenz davon, Oligonucleotiden mit einer komplementären Sequenz davon und Nucleinsäuremolekülen, umfassend mindestens einen Bereich, der eine der in SEQ ID Nr. 8 bis 135 dargestellten Nucleotidsequenzen, eine mutierte Nucleotidsequenz davon, erhältlich durch Deletion und/oder Addition von 1 bis 10 Nucleotiden und/oder Substitution von 1 bis 3 Nucleotiden einer der in SEQ ID Nr. 8 bis 135 dargestellten Nucleotidsequenzen, oder eine komplementäre Nucleotidsequenz davon und einen oder mehrere zusätzliche Bereiche mit einer Gesamtlänge von mindestens 1 Nucleotid aufweist.

11. Kit nach Anspruch 10, umfassend mindestens 24 zweite Behälter mit mindestens 24 verschiedenen Sonden zum Nachweis und/oder zur Bestimmung der HPV6-, HPV11-, HPV16-, HPV18-, HPV31-, HPV33-, HPV35h-, HPV39-, HPV40-, HPV42-, HPV43-, HPV44-, HPV45-, HPV51-, HPV52-, HPV53-, HPV56-, HPV58-, HPV59-, HPV66-, HPV68-, HPV70-, HPV73- und HPV82-Genotypen, wobei jeder Behälter mindestens eine Sonde enthält und wobei alle in einem Behälter enthaltenen Sonden nur einen spezifischen genitalen HPV-Genotyp nachweisen können.

12. Kit zum Nachweis und/oder zur Bestimmung genitaler HPV-Genotypen, umfassend mindestens einen ersten Behälter mit mindestens einem Primer zur Amplifikation von Bereichen des HPV-Gens E1, ausgewählt aus Oligonucleotiden nach einem der Ansprüche 1 oder 2, einem Gemisch aus Oligonucleotiden nach Anspruch 3 und/oder Primer-Paaren nach Anspruch 4 oder 5, und einen Nucleotid-Array zum Nachweis und/oder zur Bestimmung des Genotyps eines in einer biologischen Probe enthaltenen humanen Papillomavirus, umfassend einen festen Träger mit einer Oberfläche und mindestens ein an die Träger-Oberfläche gebundenes erstes Oligonucleotid oder Nucleinsäuremolekül, das als Sonde zur Untersuchung des HPV-Gens E1 oder eines Teils davon, zum Nachweis und zur Bestimmung eines genitalen humanen HPV-Genotyps geeignet ist, ausgewählt aus der Gruppe bestehend aus:
a) HPV-Genotyp-spezifischen Oligonucleotiden mit den in SEQ ID Nr. 8 bis 135 dargestellten Nucleotidsequenzen,
b) Oligonucleotiden, die eine gegenüber einem der Oligonucleotide nach a) mutierte Nucleotidsequenz aufweisen, nämlich eine Deletion oder Addition von 1 bis 10 Nucleotiden oder eine Substitution von 1 bis 3 Nucleotiden in einer der in a) dargestellten Nucleotidsequenzen,
c) Oligonucleotiden, die eine Nucleotidsequenz aufweisen, die über die gesamte Länge zu der Nucleotidsequenz eines Oligonucleotids nach a) oder b) komplementär ist,
d) Nucleinsäuremolekülen, umfassend mindestens einen Bereich, der eine der in a) bis c) dargestellten Nucleotidsequenzen und einen oder mehrere zusätzliche Bereiche mit einer Gesamtlänge von mindestens einem Nucleotid aufweist, und
e) Gemischen der Oligonucleotide nach a) bis c) und/oder der Nucleinsäuremoleküle nach d).

13. Kit nach einem der Ansprüche 10 bis 12, umfassend zwei erste Behälter, wobei ein Behälter äquimolare Mengen der Oligonucleotide mit den in SEQ ID Nr. 2 bis 6 dargestellten Nucleotidsequenzen enthält und ein Behälter ein Oligonucleotid mit der in SEQ ID Nr. 7 dargestellten Nucleotidsequenz enthält.

14. Kit nach einem der Ansprüche 10 bis 13, umfassend sechs erste Behälter, wobei fünf Behälter jeweils eines der Oligonucleotide mit den in SEQ ID Nr. 2 bis 6 dargestellten Nucleotidsequenzen enthalten und ein Behälter ein Oligonucleotid mit der in SEQ ID Nr. 7 dargestellten Nucleotidsequenz enthält.

15. Kit nach einem der Ansprüche 10 bis 14, umfassend zusätzlich einen Behälter mit einer Kontroll-Nucleinsäure, die unter Verwendung eines Oligonucleotids nach Anspruch 1 oder 2 mit einer der in SEQ ID Nr. 1 bis 6 dargestellten Nucleotidsequenzen als Forward-Primer und eines Oligonucleotids mit der in SEQ ID Nr. 7 dargestellten Nucleotidsequenz als Reverse-Primer amplifiziert werden kann.

16. Verwendung eines Oligonucleotids nach einem der Ansprüche 1 oder 2, eines Gemisches aus Oligonucleotiden nach Anspruch 3 oder eines Primer-Paares nach Anspruch 4 oder 5 zur Amplifikation eines Nucleinsäure-Bereiches eines genitalen humanen Papillomavirus.

17. Verwendung eines Oligonucleotids mit einer der in SEQ ID Nr. 1 bis 6 dargestellten Nucleotidsequenzen, eines Oligonucleotides, das eine Nucleotidsequenz aufweist, die über die gesamte Länge zu einer der in SEQ ID Nr. 1 bis 6 dargestellten Nucleotidsequenzen komplemetär ist, oder eines Primer-Paares nach einem der Ansprüche 4 oder 5 zur in vitro Diagnose und/oder Früherkennung von Krankheiten, die von genitalen humanen Papillomaviren verursacht werden.

18. Verwendung eines Oligonucleotids mit einer der in SEQ ID Nr. 1 bis 6 dargestellten Nucleotidsequenzen, eines Oligonucleotides, das eine Nucleotidsequenz aufweist, die über die gesamte Länge zu einer der in SEQ ID Nr. 1 bis 6 dargestellten Nucleotidsequenzen komplementär ist, oder eines Primer-Paares nach einem der Ansprüche 4 oder 5 zur Herstellung eines Kits oder Nucleotid-Arrays zur in vitro Diagnose von Krankheiten, die von genitalen humanen Papillomaviren verursacht werden.

19. Verwendung eines Gemisches aus Oligonucleotiden gemäß Anspruch 3, eines Gemisches aus Oligonucleotiden, die eine Nucleotidsequenz aufweisen, die über die gesamte Länge zu der in SEQ ID Nr. 7 dargestellten Nucleotidsequenz komplementär ist, zur in vitro Diagnose und/oder Früherkennung von Krankheiten, die von genitalen humanen Papillomaviren verursacht werden.

20. Verwendung eines Gemisches aus Oligonucleotiden gemäß Anspruch 3, eines Gemisches aus Oligonucleotiden, die eine Nucleotidsequenz aufweisen, die über die gesamte Länge zu der in SEQ ID Nr. 7 dargestellten Nucleotidsequenz komplementär ist, zur Herstellung eines Kits oder Nucleotid-Arrays zur in vitro Diagnose von Krankheiten, die von genitalen humanen Papillomaviren verursacht werden.

## Claims

1. The oligonucleotide that can be used as the forward primer for amplifying a nucleic acid region of a genital human papillomavirus (HPV) and having the sequence 5'-CAR GCI AAA WWW KTD AAR GAY TGT G-3 'or 5'-CAR GCN AAA WWW KTD AAR GAY TGT G-3' (SEQ ID No. 1), wherein R is A or G, W is T or A, K is T or G, I is inosine, N is A, T, G or C, D is A, T or G, and Y is C or T.

2. An oligonucleotide according to claim 1, wherein the oligonucleotide is selected from the group consisting of:
a) an oligonucleotide having the nucleotide sequence 5'-CAR GCI AAA TAT KTR AAA GAT TGT G-3' or 5'-CAR GCN AAA TAT KTR AAA GAT TGT G-3' (SEQ ID No. 2),
b) an oligonucleotide having the nucleotide sequence 5'-CAR GCA AAA TAT GTW AAG GAT TGT G-3' (SEQ ID No. 3),
c) an oligonucleotide having the nucleotide sequence 5'-CAR GCW AAA ATT GTA AAR GAT TGT G-3' (SEQ ID No. 4),
d) an oligonucleotide having the nucleotide sequence 5'-CAA GCA AAA ATA GTA AAR GAC TGT G-3' (SEQ ID No. 5), and
e) an oligonucleotide having the nucleotide sequence 5'-CAR GCA AAA TAT GTA AAA GAC TGT G-3 '(SEQ ID No. 6),
wherein R is A or G, W is T or A, K is T or G, I is inosine, and N is A, T, G or C.

3. A mixture of oligonucleotides that can be used as reverse primer for amplifying a nucleic acid region of a genital human papillomavirus, having the nucleotide sequence 5'-ARY GGY TSY ARC CAA AAR TGR CT-3' (SEQ ID No. 7), wherein R is A or G, Y is C or T and S is C or G.

4. A primer pair for amplifying a nucleic acid region of a genital human papillomavirus (HPV), comprising a forward primer and a reverse primer, wherein said forward primer is selected from the group consisting of:
a) an oligonucleotide according to claim 1 or 2 having one of the nucleotide sequences represented by SEQ ID Nos. 1 to 6, and
b) a mixture of the oligonucleotides of a),
and the reverse primer is the oligonucleotide having the nucleotide sequence represented by SEQ ID No. 7.

5. The primer pair according to claim 4, wherein the forward primer is an equimolar mixture of the oligonucleotides having the nucleotide sequences represented by SEQ ID Nos. 2 to 6, and the reverse primer is the oligonucleotide having the nucleotide sequence represented by SEQ ID No. 7.

6. A method of amplifying a region of a nucleic acid of a genital human papillomavirus present in a biological sample, comprising performing a nucleic acid amplification method using a primer pair comprising a forward primer and a reverse primer, wherein the forward primer is selected from the group consisting of:
a) an oligonucleotide according to claim 1 or 2 having one of the nucleotide sequences represented by SEQ ID Nos. 1 to 6, and
b) a mixture of the oligonucleotides of a),
and the reverse primer is the oligonucleotide having the nucleotide sequence represented by SEQ ID No. 7.

7. The method according to claim 6, wherein the nucleic acid amplification method is a PCR (polymerase chain reaction) method, and said nucleic acid amplification is performed under the following temperature conditions:
a) heating to 95 °C, increasing the temperature by 1 °C per sec,
b) maintaining the temperature at 95 °C for 10 min,
c) performing 40 cycles, each comprising 30 seconds at 95 °C, 30 sec at 55 °C and 1 min at 72 °C,
d) maintaining the temperature at 72 °C for 5 min, and
e) cooling to 4 °C.

8. The method according to any one of claims 6 or 7, wherein the amplification product being provided with a marker during or after the amplification reaction.

9. A method for detecting and/or identifying a genital HPV genotype, comprising analyzing the HPV E1 gene present in a biological sample, or a part thereof, of a genital human papillomavirus by hybridization with at least one probe, wherein the probe is selected from the group consisting of:
a) HPV genotype-specific oligonucleotides having the nucleotide sequences represented by SEQ ID Nos. 8 to 135,
b) oligonucleotides having a mutated nucleotide sequence compared to one of the oligonucleotides of a), namely, a deletion or addition of 1 to 10 nucleotides or a substitution of 1 to 3 nucleotides in one of the nucleotide sequences represented in a),
c) oligonucleotides that have a nucleotide sequence that is complementary over the entire length to the nucleotide sequence of an oligonucleotide of a) or b),
d) nucleic acid molecules, comprising at least one region having one of the nucleotide sequences represented in a) to c) and one or more additional regions having a total length of at least one nucleotide, and
e) mixtures of the oligonucleotides of to a) to c) and/or the nucleic acid molecules of d), and detecting the hybridization, and wherein, prior to the hybridization with the probe, the HPV nucleic acid present in the biological sample is amplified, and the amplification of the nucleic acid is carried out by a method according to any one of claims 6 to 8.

10. A kit for detecting and/or identifying genital HPV genotypes, comprising at least one first container with at least one primer for amplification of regions of the HPV E1 gene, selected from oligonucleotides according to any one of claims 1 or 2, a mixture of oligonucleotides according to claim 3 and/or primers pairs according to claim 4 or 5, and at least one second container with at least one probe for the detection of an amplified region of the HPV E1 gene, selected from oligonucleotides having a nucleotide sequences represented by SEQ ID Nos. 8 to 135, having a mutated sequence thereof, oligonucleotides having a complementary sequence thereof, and nucleic acid molecules comprising at least one region having one of the nucleotide sequences represented by SEQ ID Nos. 8 to 135, a mutated nucleotide sequence thereof, obtainable by deletion and/or addition of 1 to 10 nucleotides, and/or substitution of 1 to 3 nucleotides in one of the nucleotide sequences represented by SEQ ID Nos. 8 to 135, or a complementary nucleotide sequence thereof, and one or more additional regions having a total length of at least one nucleotide.

11. The kit according to claim 10, comprising at least 24 second containers with at least 24 different probes for detecting and/or identifying the HPV6, HPV11, HPV16, HPV18, HPV31, HPV33, HPV35h, HPV39, HPV40, HPV42, HPV43, HPV44, HPV45, HPV51, HPV52, HPV53, HPV56, HPV58, HPV59, HPV66, HPV68, HPV70, HPV73, and HPV82 genotypes, wherein each container contains at least one probe, and wherein all probes contained in a container can detect only one specific genital HPV genotype.

12. A kit for detecting and/or identifying genital HPV genotypes, comprising at least one first container with at least one primer for amplifying regions of the HPV E1 gene, selected from oligonucleotides according to any one of claims 1 or 2, a mixture of oligonucleotides according to claim 3 and/or primer pairs according to claim 4 or 5, and a nucleotide array for detecting and/or identifying the genotype of a human papillomavirus contained in a biological sample, comprising a solid support with a surface and at least one oligonucleotide or nucleic acid molecule bound to the support surface that is suitable as a probe in the analysis of the HPV E1 gene or a portion thereof for detecting and identifying a genital human HPV genotype, selected from the group consisting of:
a) HPV genotype-specific oligonucleotides having the nucleotide sequences represented by SEQ ID Nos. 8 to 135,
b) oligonucleotides having a mutated nucleotide sequence compared to one of the oligonucleotides of a), namely, a deletion or addition of 1 to 10 nucleotides or a substitution of 1 to 3 nucleotides in one of the nucleotide sequences represented in a),
c) oligonucleotides that have a nucleotide sequence that is complementary over the entire length to the nucleotide sequence of an oligonucleotide of a) or b),
d) nucleic acid molecules, comprising at least one region having one of the nucleotide sequences represented in a) to c) and one or more additional regions having a total length of at least one nucleotide, and
e) mixtures of the oligonucleotides of to a) to c) and/or the nucleic acid molecules of d),

13. The kit according to any one of claims 10 to 12, comprising two first containers, wherein one container contains equimolar quantities of the oligonucleotides having the nucleotide sequences represented by SEQ ID Nos. 2 to 6, and one container contains an oligonucleotide having the nucleotide sequence represented by SEQ ID No. 7.

14. The kit according to any one of claims 10 to 13, comprising six first containers, wherein five containers each contain one of the oligonucleotides having the nucleotide sequences represented by SEQ ID No. 2 to 6, and one container contains one oligonucleotide having the nucleotide sequence represented by SEQ ID No. 7.

15. The kit according to any one of claims 10 to 14, comprising additionally a container with a control nucleic acid, which can be amplified using an oligonucleotide according to claim 1 or 2 having one of the nucleotide sequences represented by SEQ ID Nos. 1 to 6 as a forward primer, and an oligonucleotide having the nucleotide sequence represented by SEQ ID No. 7 as a reverse primer.

16. Use of an oligonucleotide according to any one of claims 1 or 2, of a mixture of oligonucleotides according to claim 3, or of a primer pair according to claim 4 or 5 for amplifying a nucleic acid region of a genital human papillomavirus.

17. Use of an oligonucleotide having one of the nucleotide sequences represented by SEQ ID No. 1 to 6, of an oligonucleotide having a nucleotide sequence that is complementary over the entire length to one of the nucleotide sequences represented by SEQ ID Nos. 1 to 6, or of a primer pair according to any one of claims 4 or 5 for the *in vitro* diagnosis and/or early detection of diseases caused by genital human papillomaviruses.

18. Use of an oligonucleotide having one of the nucleotide sequences represented by SEQ ID No. 1 to 6, of an oligonucleotide having a nucleotide sequence that is complementary over the entire length to one of the nucleotide sequences represented by SEQ ID Nos. 1 to 6, or of a primer pair according to any one of claims 4 or 5 for producing a kit or nucleotide array for the *in vitro* diagnosis of diseases that are caused by genital human papillomaviruses.

19. Use of a mixture of oligonucleotides according to claim 3, of a mixture of oligonucleotides having a nucleotide sequence that is complementary over the entire length to the nucleotide sequence represented by SEQ ID No. 7, for the *in vitro* diagnosis and/or early detection of diseases caused by genital human papillomaviruses.

20. Use of a mixture of oligonucleotides according to claim 3, of a mixture of oligonucleotides having a nucleotide sequence that is complementary over the entire length to the nucleotide sequence represented by SEQ ID No. 7, for producing a kit or nucleotide array for the *in vitro* diagnosis of diseases that are caused by genital human papillomaviruses.

## Revendications

1. Oligonucléotide qui peut être utilisé comme amorce sens pour l'amplification d'une région d'acide nucléique d'un papillomavirus humain génital (HPV) et qui présente la séquence 5'-CAR GCI AAA WWW KTD AAR GAY TGT G-3' ou 5'-CAR GCN AAA WWW KTD AAR GAY TGT G-3' (SEQ ID N° 1), dans lequel R est A ou G, W est T ou A, K est T ou G, I est inosine, N est A, T, G ou C, D est A, T ou G et Y est C ou T.

2. Oligonucléotide selon la revendication 1, dans lequel l'oligonucléotide est sélectionné parmi le groupe constitué de :
a) un oligonucléotide avec la séquence nucléotidique 5'-CAR GCI AAA TAT KTR AAA GAT TGT G-3' ou 5'-CAR GCN AAA TAT KTR AAA GAT TGT G-3' (SEQ ID N° 2),
b) un oligonucléotide avec la séquence nucléotidique 5'-CAR GCA AAA TAT GTW AAG GAT TGT G-3' (SEQ ID N° 3),
c) un oligonucléotide avec la séquence nucléotidique 5'-CAR GCW AAA ATT GTA AAR GAT TGT G-3' (SEQ ID N° 4),
d) un oligonucléotide avec la séquence nucléotidique 5'-CAA GCA AAA ATA GTA AAR GAC TGT G-3' (SEQ ID N° 5), et
e) un oligonucléotide avec la séquence nucléotidique 5'-CAR GCA AAA TAT GTA AAA GAC TGT G-3' (SEQ ID N° 6),
dans lequel R est A ou G, W est T ou A, K est T ou G, I est inosine et N est A, T, G ou C.

3. Mélange d'oligonucléotides qui peut être utilisé comme amorce anti-sens pour l'amplification d'une région d'acide nucléique d'un papillomavirus humain génital, avec la séquence nucléotidique 5'-ARY GGY TSY ARC CAA AAR TGR CT-3' (SEQ ID N° 7), dans lequel R est A ou G, Y est C ou T et S est C ou G.

4. Paire d'amorces pour l'amplification d'une région d'acide nucléique d'un papillomavirus humain génital (HPV), comprenant une amorce sens et une amorce anti-sens, dans laquelle l'amorce sens est sélectionnée parmi le groupe constitué de :
a) un oligonucléotide selon la revendication 1 ou 2 avec une des séquences nucléotidiques représentées dans SEQ ID N° 1 à 6, et
b) un mélange des oligonucléotides selon a),
et l'amorce anti-sens est l'oligonucléotide avec la séquence nucléotidique représentée dans SEQ ID N° 7.

5. Paire d'amorces selon la revendication 4, dans laquelle l'amorce sens est un mélange équimolaire des oligonucléotides avec les séquences nucléotidiques représentées dans SEQ ID N° 2 à 6 et l'amorce anti-sens est l'oligonucléotide avec la séquence nucléotidique représentée dans SEQ ID N° 7.

6. Procédé d'amplification d'une région d'un acide nucléique présent dans un échantillon biologique d'un papillomavirus humain génital, comprenant la réalisation d'un procédé d'amplification d'acide nucléique en utilisant une paire d'amorces comprenant une amorce sens et une amorce anti-sens, dans lequel l'amorce sens est sélectionnée parmi le groupe constitué de :
a) un oligonucléotide selon la revendication 1 ou 2 avec une des séquences nucléotidiques représentées dans SEQ ID N° 1 à 6, et
b) un mélange des oligonucléotides selon a),
et l'amorce anti-sens est l'oligonucléotide avec la séquence nucléotidique représentée dans SEQ ID N° 7.

7. Procédé selon la revendication 6, dans lequel le procédé d'amplification d'acide nucléique est un procédé par PCR (réaction en chaîne par polymérase) et l'amplification d'acide nucléique est réalisée dans les conditions de température suivantes :
a) chauffage à 95°C, dans lequel la température par seconde est augmentée de 1°C,
b) maintien de la température à 95°C pendant 10 min,
c) réalisation de 40 cycles, comprenant chacun 30 secondes à 95°C, 30 secondes à 55°C et 1 min à 72°C,
d) maintien de la température à 72°C pendant 5 min et
e) refroidissement à 4°C.

8. Procédé selon une des revendications 6 ou 7, dans lequel le produit d'amplification est pourvu d'un marquage pendant ou après la réaction d'amplification.

9. Procédé de détection et/ou de détermination d'un génotype de HPV génital, comprenant la recherche du gène de HPV E1 présent dans un échantillon biologique ou d'une partie de celui-ci d'un papillomavirus humain génital par hybridation avec au moins une sonde, dans lequel la sonde est sélectionnée parmi le groupe constitué de :
a) oligonucléotides spécifiques au génotype de HPV avec les séquences nucléotidiques représentées dans SEQ ID N° 8 à 135,
b) oligonucléotides qui présentent une séquence nucléotidique mutée par rapport à un des oligonucléotides selon a), à savoir une délétion ou addition de 1 à 10 nucléotides ou une substitution de 1 à 3 nucléotides dans une des séquences nucléotidiques représentées en a),
c) oligonucléotides qui présentent une séquence nucléotidique qui est complémentaire sur la longueur entière à la séquence nucléotidique d'un oligonucléotide selon a) ou b),
d) molécules d'acide nucléique comprenant au moins une région qui présente une des séquences nucléotidiques représentées en a) à c) et une ou plusieurs régions supplémentaires avec une longueur totale d'au moins un nucléotide, et
e) mélanges des oligonucléotides selon a) à c) et/ou des molécules d'acide nucléique selon d),
et la détection de l'hybridation, et dans lequel l'acide nucléique de HPV présent dans l'échantillon biologique est amplifié avec la sonde avant l'hybridation et l'amplification de l'acide nucléique s'effectue au moyen d'un procédé selon une des revendications 6 à 8.

10. Kit de détection et/ou de détermination de génotypes de HPV génital, comprenant au moins un premier récipient avec au moins une amorce pour l'amplification de régions du gène de HPV E1, sélectionnée parmi des oligonucléotides selon une des revendications 1 ou 2, un mélange d'oligonucléotides selon la revendication 3 et/ou des paires d'amorces selon la revendication 4 ou 5, et au moins un second récipient avec au moins une sonde pour la détection d'une région amplifiée du gène de HPV E1, sélectionnée parmi des oligonucléotides avec une des séquences nucléotidiques représentées dans SEQ ID N° 8 à 135, avec une séquence mutée de celle-ci, des oligonucléotides avec une séquence complémentaire de celle-ci et des molécules d'acide nucléique comprenant au moins une région qui présente une des séquences nucléotidiques représentées dans SEQ ID N° 8 à 135, une séquence nucléotidique mutée de celle-ci, pouvant être obtenue par délétion et/ou addition de 1 à 10 nucléotides et/ou substitution de 1 à 3 nucléotides d'une des séquences nucléotidiques représentées dans SEQ ID N° 8 à 135, ou une séquence nucléotidique complémentaire de celle-ci et une ou plusieurs régions supplémentaires avec une longueur totale d'au moins 1 nucléotide.

11. Kit selon la revendication 10, comprenant au moins 24 seconds récipients avec au moins 24 sondes différentes pour la détection et/ou pour la détermination des génotypes de HPV6, HPV11, HPV16, HPV18, HPV31, HPV33, HPV35h, HPV39, HPV40, HPV42, HPV43, HPV44, HPV45, HPV51, HPV52, HPV53, HPV56, HPV58, HPV59, HPV66, HPV68, HPV70, HPV73 et HPV82, dans lequel chaque récipient contient au moins une sonde et dans lequel toutes les sondes contenues dans un récipient peuvent uniquement détecter un génotype de HPV génital spécifique.

12. Kit de détection et/ou de détermination de génotypes de HPV génital, comprenant au moins un premier récipient avec au moins une amorce pour l'amplification de régions du gène de HPV E1, sélectionnée parmi des oligonucléotides selon une des revendications 1 ou 2, un mélange d'oligonucléotides selon la revendication 3 et/ou des paires d'amorces selon la revendication 4 ou 5, et un ensemble nucléotidique pour la détection et/ou pour la détermination du génotype d'un papillomavirus humain contenu dans un échantillon biologique, comprenant un support fixe avec une surface et au moins un premier oligonucléotide ou une première molécule d'acide nucléique fixé(e) à la surface du support qui convient comme sonde à la recherche du gène de HPV E1 ou d'une partie de celui-ci, à la détection et à la détermination d'un génotype de HPV humain génital, sélectionné(e) parmi le groupe constitué de :
a) oligonucléotides spécifiques au génotype de HPV avec les séquences nucléotidiques représentées dans SEQ ID N° 8 à 135,
b) oligonucléotides qui présentent une séquence nucléotidique mutée par rapport à un des oligonucléotides selon a), à savoir une délétion ou addition de 1 à 10 nucléotides ou une substitution de 1 à 3 nucléotides dans une des séquences nucléotidiques représentées en a),
c) oligonucléotides qui présentent une séquence nucléotidique qui est complémentaire sur la longueur entière à la séquence nucléotidique d'un oligonucléotide selon a) ou b),
d) molécules d'acide nucléique comprenant au moins une région qui présente une des séquences nucléotidiques représentées en a) à c) et une ou plusieurs régions supplémentaires avec une longueur totale d'au moins un nucléotide, et
e) mélanges des oligonucléotides selon a) à c) et/ou des molécules d'acide nucléique selon d).

13. Kit selon une des revendications 10 à 12, comprenant deux premiers récipients, dans lequel un récipient contient des quantités équimolaires des oligonucléotides avec les séquences nucléotidiques représentées dans SEQ ID N° 2 à 6 et un récipient contient un oligonucléotide avec la séquence nucléotidique représentée dans SEQ ID N° 7.

14. Kit selon une des revendications 10 à 13, comprenant six premiers récipients, dans lequel cinq récipients contiennent chacun un des oligonucléotides avec les séquences nucléotidiques représentées dans SEQ ID N° 2 à 6 et un récipient contient un oligonucléotide avec la séquence nucléotidique représentée dans SEQ ID N° 7.

15. Kit selon une des revendications 10 à 14, comprenant en outre un récipient avec un acide nucléique témoin qui peut être amplifié en utilisant un oligonucléotide selon la revendication 1 ou 2 avec une des séquences nucléotidiques représentées dans SEQ ID N° 1 à 6 comme amorce sens et un oligonucléotide avec la séquence nucléotidique représentée dans SEQ ID N° 7 comme amorce anti-sens.

16. Utilisation d'un oligonucléotide selon une des revendications 1 ou 2, d'un mélange d'oligonucléotides selon la revendication 3 ou d'une paire d'amorces selon la revendication 4 ou 5 pour l'amplification d'une région d'acide nucléique d'un papillomavirus humain génital.

17. Utilisation d'un oligonucléotide avec une des séquences nucléotidiques représentées dans SEQ ID N° 1 à 6, d'un oligonucléotide qui présente une séquence nucléotidique qui est complémentaire sur la longueur entière à une des séquences nucléotidiques représentées dans SEQ ID N° 1 à 6, ou d'une paire d'amorces selon une des revendications 4 ou 5 pour le diagnostic et/ou dépistage précoce in vitro de maladies qui sont provoquées par des papillomavirus humains génitaux.

18. Utilisation d'un oligonucléotide avec une des séquences nucléotidiques représentées dans SEQ ID N° 1 à 6, d'un oligonucléotide qui présente une séquence nucléotidique qui est complémentaire sur la longueur entière à une des séquences nucléotidiques représentées dans SEQ ID N° 1 à 6, ou d'une paire d'amorces selon une des revendications 4 ou 5 pour la fabrication d'un kit ou ensemble nucléotidique pour le diagnostic in vitro de maladies qui sont provoquées par des papillomavirus humains génitaux.

19. Utilisation d'un mélange d'oligonucléotides selon la revendication 3, d'un mélange d'oligonucléotides qui présentent une séquence nucléotidique qui est complémentaire sur la longueur entière à la séquence nucléotidique représentée dans SEQ ID N° 7 pour le diagnostic et/ou dépistage précoce in vitro de maladies qui sont provoquées par des papillomavirus humains génitaux.

20. Utilisation d'un mélange d'oligonucléotides selon la revendication 3, d'un mélange d'oligonucléotides qui présentent une séquence nucléotidique qui est complémentaire sur la longueur entière à la séquence nucléotidique représentée dans SEQ ID N° 7 pour la fabrication d'un kit ou ensemble nucléotidique pour le diagnostic in vitro de maladies qui sont provoquées par des papillomavirus humains génitaux.
